# EUROPEAN PATENT SPECIFICATION

(11) **EP 3 707 241 B1**
(45) Date of publication and mention of the grant of the patent: **22.04.2026**
(21) Application number: 18876269.4
(22) Date of filing: 09.11.2018
(51) Int. Cl.: C12N 5/07, C12N 5/076, G01N 15/00, A61D 19/00, A61D 19/02, C12Q 1/68, C12Q 1/6879, A61K 35/52

(54) **SEXED SPERM BULK SEPARATION SYSTEMS**
SYSTEME ZUR MASSENTRENNUNG VON GESEXTEM SPERMA
SYSTÈMES DE SÉPARATION EN VRAC DE SPERME SEXUÉ

(30) Priority: 10.11.2017 US 201762584598 P
(43) Date of publication of application: 16.09.2020
(73) Proprietor: Cytolutions, LLC, Columbus, OH 43212 (US)
(72) Inventor: KRUG, Kristie, Upper Arlington OH 43220 (US)
(74) Representative: Kador & Partner Part mbB
(86) International application number: PCT/US2018/060178
(87) International publication number: WO 2019/094831

(56) References cited:
- WO-A1-2014/035840
- WO-A2-2005/042721
- US-A1- 2006 067 916
- US-A1- 2016 091 410
- CHAN P J ET AL: "A simple zeta method for sperm selection based on membrane charge", FERTILITY AND STERILITY, ELSEVIER, AMSTERDAM, NL, vol. 85, no. 2, 1 February 2006 (2006-02-01), pages 481 - 486, XP028061881, ISSN: 0015-0282, [retrieved on 20060201], DOI: 10.1016/J.FERTNSTERT.2005.07.1302
- NASR ESFAHANI MOHAMMAD HOSSEIN ET AL: "Zeta Sperm Selection Improves Pregnancy Rate and Alters Sex Ratio in Male Factor Infertility Patients: A Double-Blind, Randomized Clinical Trial", INTERNATIONAL JOURNAL OF FERTILITY & STERILITY, 1 July 2016 (2016-07-01), Iran, pages 253 - 260, XP055824035, Retrieved from the Internet <URL:https://pdfs.semanticscholar.org/b53f/568022d0bc783a6412ffbb5f75cbd7c24c25.pdf> [retrieved on 20210713], DOI: 10.22074/ijfs.2016.4917
- BARAKAT IBRAHIM A. H. ET AL: "Effect of Various Concentrations of Caffeine, Pentoxifylline, and Kallikrein on Hyperactivation of Frozen Bovine Semen", vol. 2015, 1 January 2015 (2015-01-01), pages 1 - 7, XP055824049, ISSN: 2314-6133, Retrieved from the Internet <URL:https://www.ncbi.nlm.nih.gov/pmc/articles/PMC4407405/pdf/BMRI2015-948575.pdf> DOI: 10.1155/2015/948575
- AINSWORTH C J ET AL: "The electrophoretic separation of spermatozoa: an analysis of genotype, surface carbohydrate composition and potential for capacitation", INTERNATIONAL JOURNAL OF ANDROLOGY,, vol. 34, no. 5, Pt.2, 1 October 2011 (2011-10-01), pages e422 - e434, XP009172140, DOI: 10.1111/J.1365-2605.2011.01164.X
- HENKEL RALF: "Sperm preparation: state-of-the-art--physiological aspects and application of advanced sperm preparation methods", ASIAN JOURNAL OF ANDROLOGY, NATURE PUBLISHING GROUP, US, vol. 14, no. 2, 1 March 2012 (2012-03-01), pages 260 - 269, XP009172146, ISSN: 1008-682X, DOI: 10.1038/AJA.2011.133
- DOM�NGUEZ ESTEBAN ET AL: "Sperm Sexing Mediated by Magnetic Nanoparticles in Donkeys, a Preliminary In Vitro Study", vol. 65, 1 June 2018 (2018-06-01), US, pages 123 - 127, XP055824036, ISSN: 0737-0806, Retrieved from the Internet <URL:https://www.researchgate.net/profile/Henry-Clemente/publication/324857446_Sperm_Sexing_Mediated_by_Magnetic_Nanoparticles_in_Donkeys_a_Preliminary_In_Vitro_Study/links/5b1969bcaca272021ceef620/Sperm-Sexing-Mediated-by-Magnetic-Nanoparticles-in-Donkeys-a-Preliminary-In-Vitro-Study.pdf> DOI: 10.1016/j.jevs.2018.04.005
- PARRISH, J.J. ET AL.: "Capacitation of Bovine Sperm by Heparin", BIOLOGY OF REPRODUCTION, vol. 38, 1 June 1988 (1988-06-01), pages 1171 - 1180, XP055147210, DOI: doi:10.1095/biolreprod38.5.1171

## Description

### Technical Field

This invention relates generally to the field of sex selection of sperm such as is useful in producing offspring of a desired gender. It relates to the selection of sperm using sex-based characteristics and can even apply beyond such applications. In some specifics, it involves processes whereby sex selection of sperm can occur through bulk processing of sperm. In one embodiment it can involve the use of magnetic modalities for cellular identification and separation such as for sex selection of sperm.

### Background

The selection of sperm based on sex-related characteristics is an area that has become well developed through a particular technology. It is a field that developed largely as a result of the invention disclosed in 1992 in US Pat. No. 5135759 to Johnson, et al. from work at the US Department of Agriculture. The Johnson patent explained the ability to utilize a particular DNA staining dye, Hoechst 33342, to individually discern DNA quantity and therefore exploit that fact that X and Y chromosome-bearing sperm have differing DNA content. As that patent noted, the differences were 3.4% in boar, 3.8% in bull, and 4.2% in ram sperm, and these differences could be detected by a flow cytometer with the individual cells then separated by the flow cytometer to yield X and Y sperm sample with over 80% purity. This individual cell detection-based process has been improved over the years but it still remains the only practically usable way in which such results are achieved. The sex selection of sperm is mostly done using a fluorescent dye that labels the DNA content and thus disparity of the sperm and individual cells are then subsequently sorted into X and Y populations of sperm using flow-based cytometry or the like. Although some such processes can yield greater than 90% purity in each sorted fraction, individual cell-based processes such as the flow cytometry sorting of sperm is inefficient and time consuming. It can also result in greatly damaged sperm due to sheer stresses inherent in a flow cytometer, the isolation of the individual cells for individual analysis, possibly the staining of the cells, and the like. These effects can be significant especially for sperm cells because they can be considered from some perspectives as more fragile cells where the desired functioning (indeed, fertilization is the desired function for a sperm cell) can be adversely impacted by large variety of conditions, treatments, or environments. In this regard, the present invention offers advantages that can be especially noticed in maintaining the viability and suitability for their desired purpose when used in practical and real-world processes that can exist such as in an agricultural and ranching setting as is often the case.

Interestingly, a bulk sex selection separation process that can occur in a matter of minutes, separate larger numbers of cells, and is more gentle on the cellular structure has been desired for years. However, this has not been able to be practically, or perhaps repeatedly, achieved even when known and studied processes and techniques were applied. For example, over twenty years ago, it was suggested by Parrish et al. that a bulk separation of bovine sperm from plasma could be achieved by either swim-up or Percoll gradient separation methods. Theriogenology 1995; 44:859-69. Expansion of this technique and application to regular sperm processing did not occur however. In fact, studies, such as a study by Roelf in 1992, and the fact that years on no techniques other than flow cytometry exist are stark testimony to the fact that such proposals were never enabled. And, at this point in time, it is simply true that no practical and repeatable alternative ways of achieving sex-based separation other than flow cytometry exist. The long-desired bulk sex-based characteristic separation for sperm has just continued to remained unavailable.

For example, in 2009 Machado et al. proposed a simple use of the DNA differences as a reason for differences in weight and density as a grounds for the desired bulk separation. Theriogenology 71:1289-97, 2009. However, while it was postulated that the known difference in DNA mass led to a difference in weight and density between X and Y chromosome bearing sperm, this may not have been correct because it did not result in a repeatable or practical bulk sex selection separation process. In fact, one of the issues that may have led to faulty conclusion may have been the type of tests used to determine if there were, indeed, different sex chromosome bearing sperm in the result. At one time, testing for an F-body was deemed indicative of the sex chromosome of the particular sperm cells. As references subsequently explained, this was either not correct or did not yield repeatable results and so often times what was believed to be an enabling disclosure of a repeatable, actually existing effect was not proven to be true either over time, by subsequent implementation for what the alleged process promised, or through subsequent attempts at verification and/or testing of the alleged procedure.

Whether applied to a discontinuous or continuous gradient, or whether used with application of Percoll density gradient centrifugation, the ability to use these technologies did not result in skewing the outcome of the X or Y sperm. Even though motility was increased, the ratios of X bearing and Y bearing sperm remained as a 1:1 ratio. Thus, in even an attempt to apply the Percoll gradient separation technique over a decade after it was previously considered, there was no actionable differential. Thus, at that time, it was concluded that Percoll use for the separation of sperm X bearing chromosomes from Y bearing chromosomes based on weight and density could not be used as it did not alter the 1:1 ratio of X to Y cells from an ejaculate.

Even the application of other bulk processing technologies to sperm themselves have not led to a bulk sex characteristic separation process. For example, the use of electrical charge, zeta charge, and even magnetic particles and/or molecules for certain biological processes or application has been known for at least some years. As disclosed in US Patent Publication US2012/0270204 to Fox et al. in 2012 the magnetic particle technique was even applied to achieve a pre-cytometer sperm sorting treatment to remove dead sperm prior to using a flow cytometer for sex selection. As this reference taught, the expectation was not to use that process for bulk sex selection, but rather to use it as a pretreatment eliminate dead or dying sperm so that the age-old flow cytometer individual cell separation process could be more efficiently achieved. Similar pretreatment was the anticipated goal and all that was indeed achieved by subsequent efforts such as those by the present inventor in US Patent Publication US2014/0234864 to Krug as there was then no known way to improve such processes to achieve sex selection of sperm. Even the general application of electric charge characteristics by Chan et al. over a decade ago in 2006 to evaluate and remove all sperm (i.e., regardless of sex characteristic) having poor DNA integrity did not provide those skilled in the art any ability to achieve a bulk sex separation process as even the use involving sperm DNA itself did not lead to any ability to use that or even the known difference in total DNA content as some type of bulk sex selection modality.

### Disclosure of Invention

Accordingly, the present invention provides a method whereby sperm can be practically and repeatedly separated in bulk, based on a sex characteristic as generally defined in claim 1. The overall invention of a bulk sex selection process is presented in a manner that includes a variety of aspects or embodiments which may be applied in different manners with differing values or attributes and in differing combinations to suit the needs of the user and as may be optimal for any type of application. At one level the invention presents a process whereby sperm can be induced to exhibit an attribute that differs between X and Y bearing sperm. This attribute can be used perhaps at the optimal time or in an optimal manner to allow one type of sperm to be preferentially selected and then separated from the other.
Thus, the invention provides a method that may allow faster separation processes as compared to the speed with which individual cell identification-based processes are achieved.

Another object of the invention may be to provide technologies whereby sperm may not be subjected to harsh or damaging environments so sperm separated can be more viable and perhaps more useful for their desired processes such as insemination.

Yet another object of the invention may be to provide a sex selection process that has the ability to quickly and reliably achieve higher separation purity than existing processes.

Still another object of the invention may be to provide sex selection processes that may be less expensive and more easily achieved with less reliance on needs for expensive or complex equipment or highly trained equipment operators.

Through embodiments of the present invention, it may now be possible to induce a sex-based differential change and to use this change to bulk separate sperm based on sex-related characteristics. One embodiment of the present invention may provide a method for separating X bearing sperm from Y bearing sperm in an ejaculate. Benefits and advantages of the present invention include, but are not limited to, bulk purification of one sperm population from another in a rapid, gentle process. This process can result in a higher sperm number separated in a minimal amount of time with less cellular damage. In some embodiments, sperm can be sex selected magnetically based on a difference perhaps such as surface charge and perhaps such as during the capacitation process prior to fertilization.

As a specific initial embodiment to facilitate capacitation, the pH and incubation time of the sperm may be altered. Sperm that is diluted perhaps such as in TRIS buffers or left to sit at room temperature can maintain a pH range of 6.5 to 6.8 for a longer period of time. A capacitation media can be applied to induce capacitation or changes that may be associated with capacitation.

Of course, as those skilled in the art would readily appreciate, different media can have different properties and one can potentially have more benefit than another for certain applications or certain cell species. Once capacitated, sperm may alter a property perhaps such as causing a net negative charge or neutral charge to become a slightly positive charge. Again, the cutoff time period and most effective pH or other environment to cause such a change or even to capacitate the sperm can be varied. As one example, capacitation time cutoff may be based on the number of X sperm remaining that still have good motility and viability.

Interestingly, in this embodiment, the way in which the sperm are handled or capacitation or other changes are induced can allow or cause the Y chromosome bearing sperm to achieve a change or an amount of change faster or to a greater degree than X chromosome bearing sperm and this difference can be exploited to achieve the desired bulk or other different type of separation. Importantly, these changes can be induced while leaving a number of viable X sperm available to process after removal of Y sperm. Such changes causes or uses a change in surface charge of the capacitated sperm as a differential effect for separation.

To achieve the foregoing, and in accordance with some purposes of embodiments of the present invention as broadly described herein, a method for separating X bearing sperm from Y bearing sperm may include but is not limited to: using magnetic particles with inherent zeta potential charges to bind to different subpopulations of sperm, and separating the cellular or otherwise bound magnetic particles such as in the presence of a magnetic field, a negatively charged substrate such as glass or silica, dextran, or any other substrate or effect to which the negative zeta charge reacts such as in the presence of buffers or otherwise. With reference to sperm, embodiments of the invention can include, a method for separating X bearing sperm from Y bearing sperm, hereof, including: obtaining charged particles; mixing conjugated or unconjugated, optionally charged magnetic particles with a sample of sperm; and separating the sperm bound to magnetic particles in the presence of a magnetic field.

Additional objects, advantages and novel features of the invention are set forth in part in the description which follows, and in part will become apparent to those skilled in the art upon examination of the following or may be learned by practice of the invention or adaptation of its basic principles to particular situations, cells, substances, or species.

### Brief Description of Drawings

The accompanying figures, which are incorporated in and form a part of the specification, illustrate one or more embodiments of the present invention as related to sperm, and, together with the description, serve to explain the broad general principles of the invention. In the figures:
Figure 1 illustrates a pH change in neat sperm over time at room temperature in various buffers with various pHs for a first bull.
Figure 2 illustrates a pH change in neat sperm over time at room temperature in various buffers with various pHs for a second bull.
Figure 3 illustrates a pH change in neat sperm over time at room temperature in various buffers with various pHs for a third bull.
Figure 4 illustrates a pH change in neat sperm over time at room temperature in various buffers with various pHs for a fourth bull.
Figure 5 illustrates an example of the pH value of frozen thawed purified X and Y sperm as well as conventional sperm in an Sp-TALP-H buffer over time.
Figure 6 illustrates a capacitation possibly as deduced from pH change of Y sperm in increasingly concentrated heparin buffers over time.
Figure 7 illustrates a flow cytometry histogram of Y bearing sperm for which capacitation was induced by 10ug/ml heparin for three hours.
Figure 8 illustrates a zeta potential of iron oxide nanoparticles that were mixed with Sp-TALP-H at the level of 10 ug/ml heparin.

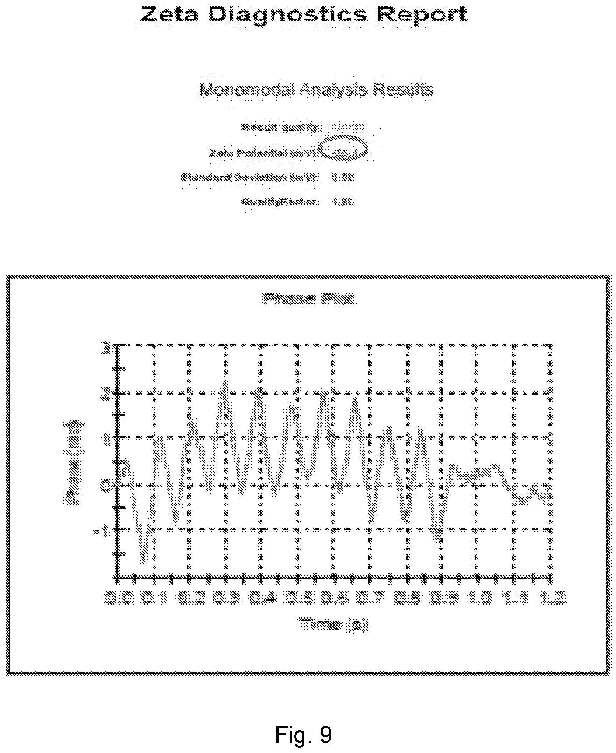

Figure 9 illustrates a zeta potential of iron oxide nanoparticles that were mixed with Sp-TALP-H at the level of 20 ug/ml heparin.

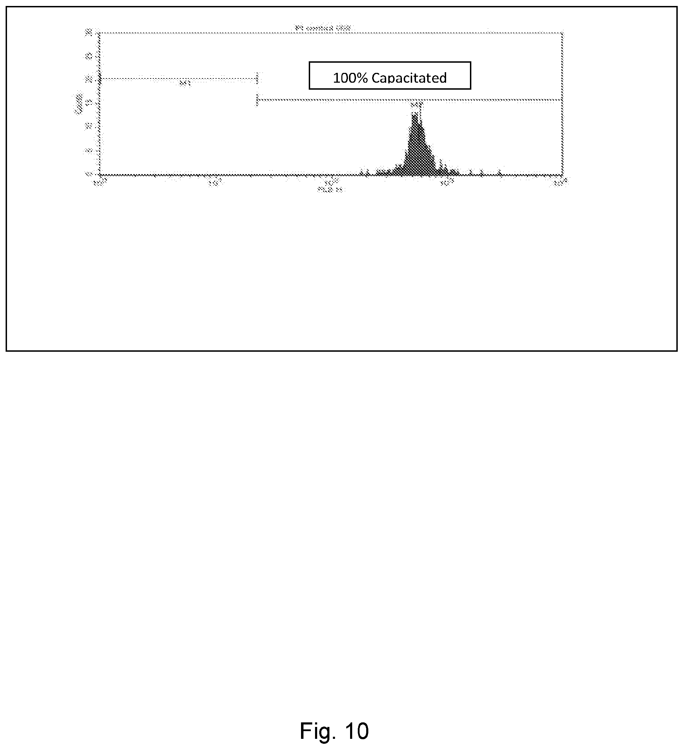

Figure 10 illustrates a flow cytometry histogram of Y bearing sperm for which capacitation was induced by 20ug/ml heparin for six hours prior to mixing with magnetic particles.

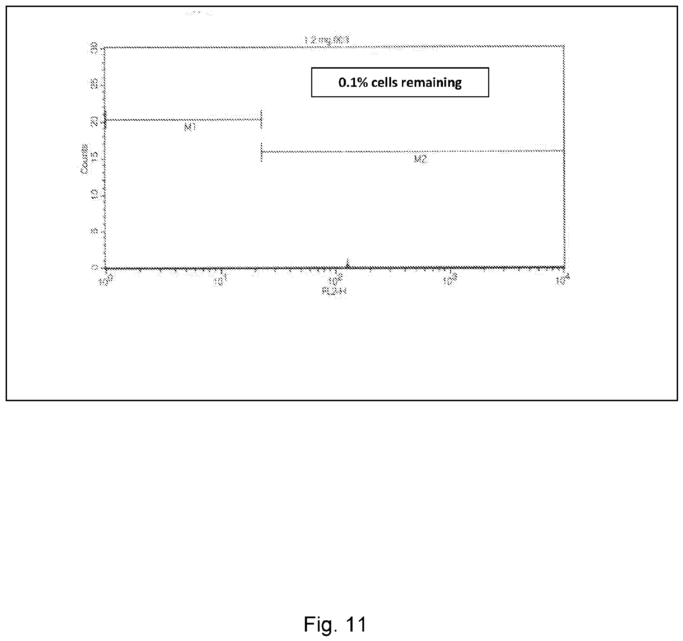

Figure 11 illustrates a flow cytometry histogram of Y bearing sperm for which capacitation was similarly induced by 20ug/ml heparin for six hours after treatment with 1.2 mg of iron nanoparticles for 20 minutes and after magnetic removal by three minutes on amagnet.

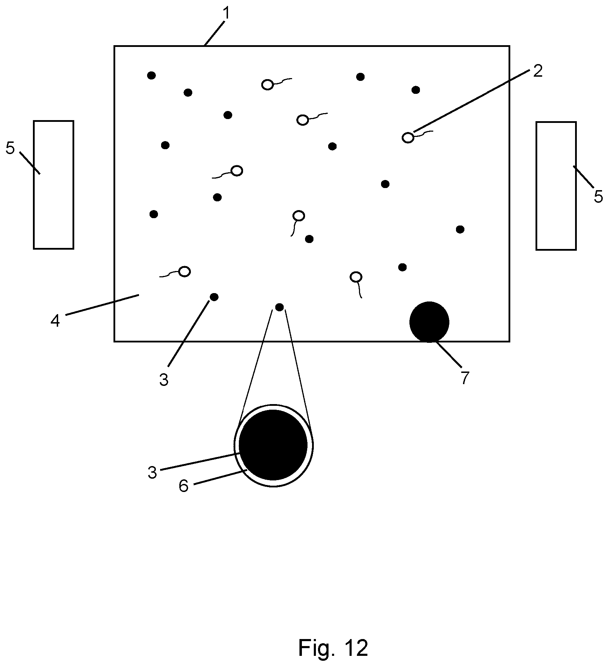

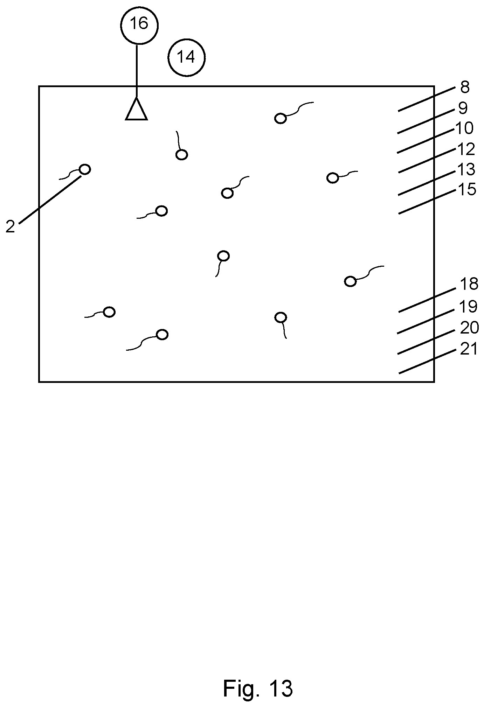

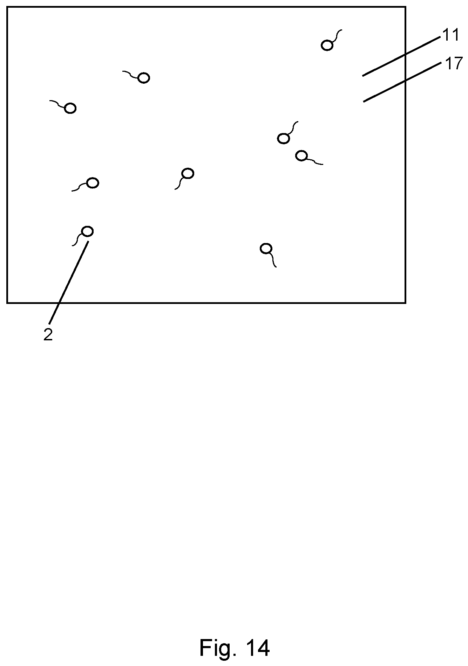

Figures 12-14 illustrate systems in schematic that include aspects as may be configured according to embodiments of the invention.

### Mode(s) for Carrying Out the Invention

As mentioned earlier, the present invention includes a variety of aspects, which may be combined in different ways and may be applied to sex selection of sperm including but not limited to in a bulk manner, or with magnetic nanoparticles. The following descriptions are provided to list elements and describe some of the embodiments of the present invention.

The invention is intended to encompass changes as can be developed to allow bulk separation as a more desirable technique for a variety of reasons ranging from cost, to cell impacts, to results achieved. With that understanding, embodiments can involve process steps such as: establishing a collection (1) of cells, such as sperm cells (2) such as may have both X-bearing sperm cells and Y-bearing sperm cells; utilizing a cell related differential effect involving differential surface charge induced in at least some of the Y-bearing sperm cells that can be applied or used to distinguish somehow between a desired type of cell and ones that are not desired (or vice versa); acting on that effect; and then generally separating some portion of the cells for later use through various types of a separation modality (5) and even a particle separation modality. Methods of the invention involve sex chromosome differentially exhibiting sperm cells.

Recognizing that a number of authors in the sperm sexing field have announced that they have achieved the long desired holy grail of a bulk sperm sex-based separation process only to have subsequently been proven wrong and to have not enabled what they suggested they did, an aspect of embodiments of the invention can be to provide repeatable, controllable processes for those in the field to practically apply. From this perspective, embodiments of the invention can include processes that can be adapted to cause, or perhaps controlling to allow or assure, that the differential effect exists precisely when desired so it can be repeatably applied. Perhaps at its simplest, this might be through a process of just affirmatively supporting the intrinsically actionable sperm cell sex chromosome related differential effect for the collection of sperm cells, or providing a differential effect affirmative support (10) such as a condition to allow thawing and transitioning in a controlled manner, an appropriate pH increasing buffer, so that it can be assured to be present when needed. This can be particularly important because the differential effect can be one of a transient nature itself. And the differential effect can come with a need to balance its effects to maintain other needed aspects such as cell viability or function or the like.

The process is desired to be highly repeatable and so embodiments can involve timing or a window of opportunity that may be critical.

Such is especially the case for sperm cells where viability is of paramount importance. Here, the aspect of possibly just allowing the differential effect to exist without significantly compromising use of any of the sperm cells for fertilization processes can be desired. Given prior announcements that were only followed by failure to prove or repeat, an important aspect of affirmatively supporting can be the act of actually supporting the differential effect such as by causing it, by giving it a favorable environment, or perhaps just by providing a transition state timer (14) or timing after a particular known event so that the entire process not only is enabled, but it works both reliably and repeatably. This can be significant for embodiments where the sex differential property of the cells such as the sperm cell sex chromosome related differential effect of sperm cells is transitory, subtly exhibited, or short-lived when considered in the window of maintaining the cells as viable or the like.

As mentioned, an aspect of embodiments of the invention can be that of utilizing the cell related differential effect so that it can be applied or used to distinguish a desired type of cell. According to the invention, it is possible to utilize the differential effect which is differential surface charge by using it to create an association through action of sperm cell associatable particles which associate with said Y-bearing sperm cells via said differential surface charge. The association may be a differential association, so that perhaps only one type of cells is associated while the other type of cells is not associated. And the modality for associating, be it a substance, surface, particles, can be differentially associatable such as when it/they are capable of establishing or having established for it/them an association with the desired or undesired type of cells as the case may be. The modality for associating, substance, surface, particles, can be used to establish a suspension, mixture, colloid, or any other type of structure in a fluid combination so it can be mobile and can act so as to be proximal to most if not all the cells and this is one-way users can achieve proximally situating a substance in the vicinity of the collection of cells. For example, by suspending or otherwise combining associationally active particles in a buffer they may be easily fluidically combined with the sex chromosome differential exhibiting sperm cells whereby there can be established a fluid combination (4) of sperm cell sex chromosome differentially associatable particles and sex chromosome differentially exhibiting sperm cells. These two types of things can then or later act so that some become associated with the desired or undesired types of cells and thus can then form a differentially associated substance, differentially associated particle, or differentially associated surface. Thus, there may be differentially associatable particles (3), or for the sperm sexing applications, there may be sperm cell sex chromosome differentially associatable particles that associate differently (perhaps under the appropriate conditions or such) based on the very nature of whether or not that particular sperm cell is an X-bearing sperm cell or a Y-bearing sperm cell.

For X-bearing sperm cells and Y-bearing sperm cells it can be significant that only one of the two types of cells are associated with the differentially associatable substance, or surface, particles. This is because the other is essentially unaffected and has none or very little of the actually or potentially negative treatments, environments, or foreign substances that may affect its natural efficacy. Thus, embodiments of the invention can involve associating only a desired portion of the collection of sperm cells with the substance, surface, or particles. As mentioned, the invention can generally involve separating some portion of the cells for later use. Separating (as is understood from the word) involves removing one from the other. Of course, it does not matter which is removed from which within the meaning of separating; one type can be simply removed from another type. Note, however, from the perspective of viability, especially of sperm cells, cells that are associated or perhaps even bound to the separation modality such as a particle may not be in a usable form and may not ultimately be considered viable even though they may have started out that way. As a result, methods can be designed to achieve capture of one or the other types of cells where possible. And it may be that the desired type of cell and ones that are not desired are reversed in some instances.

For embodiments involving a use of particles, the particles can be considered one part of the separation modality. Another part of the particle separation modality can be some external actor, perhaps including but not limited to a force or application of a force to which the sperm cell sex chromosome differentially associatable particles in the fluid combination of sperm cell sex chromosome differentially associatable particles and sex chromosome differentially exhibiting sperm cells are responsive. As explained elsewhere, where there is a force, the force can be a magnetic force, centrifugal force, or perhaps just gravity or a gravitational force. It could also be an electrostatic force or even forces such as are encountered in an electrophoresis process. Regardless of the components or nature of the separation modality, the process can involve separating at least some of the differing types of cells, perhaps such as the X-bearing sperm cells and Y-bearing sperm cells.

In practice, some embodiments of the invention can involve some combination such as the following steps: 1) obtaining a sample; 2) incubating the sample in an appropriate buffer to induce the differential effect; 3) suspending or otherwise combining associationally active particles in a similar or perhaps identical buffer; 4) mixing an aliquot of suspended particles with suspended cells at desired ratios; 5) incubating the mixture for a desired period of time; 6) subjecting the mixture to a separation modality for a set period of time; and 7) separating, perhaps by removing, a portion of the mixture so as to yield a desired resultant selected portion of the total. Further, optional steps could include, among other options including but not limited to: alteration of the aforementioned steps; washing the sample with buffer before incubating to induce the desired effect; resuspending the sample in the same buffer after incubation or perhaps in the same buffer with the addition of bovine serum albumin (BSA) of a similar substance; achieving a desired final cell concentration such as perhaps 160 M cells/ml; coordinating a particle amount with the cell amount in some measurable manner; using an unfrozen sample or perhaps thawing a frozen semen amount to obtain the sample prior to achieving separation on the thawed sample; incubating for differing time periods.

The present invention may include a method for separating X bearing sperm from Y bearing sperm through a bulk process or in otherwise new manners thereby enriching a sperm subpopulation. Briefly, an initial embodiment of the present invention can include a method for separating X bearing sperm from Y bearing sperm using magnetic selection as but one example.

The method can be applied to cells such as contained in freshly collected samples after dilution, during and after cooling, or during and after other cell or system procedures that are employed prior to cryopreservation, or to frozen/thawed cell samples. The enriched cell populations can be used for routine procedures, prior to or after other processing techniques, prior to or after shipment of samples, and prior to or after cryopreservation or other processes.

These separated samples can be used for *in vitro* fertilization, for all mammalian sperm. In one general manner, the invention can be understood as if exploiting the aspects of capacitation and zeta charge changes associated therewith in a new manner that allows or perhaps induces such changes differentially for the X- versus the Y-bearing sperm cells. The existence of a differentiation can arise such as by affirmatively supporting an intrinsically actionable effect for any type of cell, perhaps such as a sperm cell sex chromosome related differential effect for a collection of sperm cells. In keeping with the above, the differential effect can be a naturally occurring cellular process. If well predictable, this naturally occurring cellular process can occur spontaneously, but it may be most controllable if it is triggered or controlled by an outside influence perhaps such as an environmental condition, perhaps such as by affirmatively supporting a cellular process differential transition effect or providing a cellular process differential effect affirmative support, it may be caused such as by a separately supplied, perhaps well-accepted chemical influence that does not significantly impact the cells. As mentioned above, the naturally occurring cellular process can be a process that has a transient character in that it can involve a relatively short-term changing state. It can simply be a transition effect whereby cells may be transitioning from one state to another. The inventive process for sperm cells is that of a capacitation process. Of course, capacitation, a process whereby sperm cells become capable of fertilizing, is a naturally occurring process. As can be understood from the natural act of fertilization, it can be triggered or effected through environmental conditions and/or chemical inducements and both can be used when fertilization is desired. Thus, the differential effect, perhaps such as a capacitation effect, can occur as a result of triggering capacitation for a collection of sperm cells or a capacitation trigger (13).

As background, it can be understood that once capacitated, sperm can be understood as altering the net negative charge found inherently, to a neutral or slightly positive net charge. The invention can exploit this in a manner that determines a cutoff time period for such a change. This can be determined as perhaps the most effective pH change to capacitate Y chromosome bearing sperm faster or in a larger amount or higher total amount than X chromosome bearing sperm. According to the invention, this can be done while leaving a number of viable X sperm available to process after removal of Y sperm using an effect of change in surface charge of the capacitated sperm to be used for separation.
In keeping with the aspect of having a controlled, predictable, and repeatable process that is not dependent on just lucky timing which can be unrepeatable, a number of differential effects can be considered as the principles of the invention are applied to various cells, various systems, and various desires.

And again, elements can be provided such as a differential charge effect trigger or actions can be taken that might trigger a differential charge effect for a collection of cells, perhaps such as sperm cells, trigger a differential surface charge effect for a collection of cells, or even trigger a differential zeta charge effect for a collection of cells. It is also possible to induce an effect such as by not just waiting for it to occur but by very causally affirmatively acting to make the effect occur. There can thus be a differential effect inducer (12), involving processes of inducing a sperm cell surface charge differential effect,. For embodiments where the cells exist in a fluid medium, the zeta charge changes whereby a differential is exhibited can include movement to a substantially uncharged state or perhaps just movement to a low charged state.
Thus, embodiments of the invention can involve inducing a sperm cell substantially uncharged differential effect and inducing a sperm cell low charge differential effect where appropriate or provide a sperm cell substantially uncharged differential inducer or a sperm cell low charge differential inducer.

In some embodiments, an increase of the pH by at least 0.36 units can be exploited as an indication when sperm start to capacitate and/or die and this can be achieved in a manner to effect a usable change such as the charge properties of the cell, such as a zeta charge effect. To achieve these changes, a media in which the sperm are stored can contribute to this increase in pH, so can a passage of time. In example 1, a test of four bull ejaculates in four conditions were analyzed over a period of six hours as explained below.

Understanding some underlying processes involved in some embodiments and how these can be varied can contribute to an understanding of the breadth of the invention described in its initial embodiments. In the basic process for sperm cells, it can be understood that as sperm capacitate, there is a loss of sialic acid groups on the surface of the cell. Potentially involved in the differential processes for X and Y sperm as explained here, it can be appreciated that there may be a disproportionate amount of sialic acid on Y sperm as opposed to X sperm, perhaps whereas X sperm have a higher amount of sialic acid groups. Living sperm contained in certain buffers as well as in the reproductive tract can be understood as having a net negative zeta potential. Perhaps as sialic acid groups are lost such as during capacitation (the penultimate step prior to fertilization) the net zeta potential charge of the sperm may reach zero to slightly positive mV. To the degree that X sperm contain more sialic acid groups than Y sperm, the process to reach a zero to slightly positive zeta potential charge can take longer or be achieved to a more significant degree than Y sperm. Therefore, a careful titration to cleave sialic acid groups and the subsequent addition of negatively charged substrate may yield a purified X bearing sperm population.

An indication of such changes or others that can be exploited can be in the pH of the media suspending the cells. This can affect the charge of proteins comprising the cells. Proteins function as dipolar ions mainly due to the ionization of the various R groups of the amino acids, which make up their primary structure. Thus, media pH may affect their protein-protein interactions. In the example of sperm, capacitation can involve the removal of seminal coating proteins absorbed on the sperm's surface membrane, thus, changing the pH of the capacitating medium from pH 7.2 through pH 8.4 could be expected to alter the binding of these proteins to the sperm's surface and aid in effecting the desired change.

Ionic components of the culture medium can influence mammalian sperm motility, capacitation, acrosomal integrity, and a sperm's ability to penetrate an oocyte. The pH of the medium can affect the ionization of substances within it, including proteins intrinsic to the sperm membrane and extrinsic, absorbed seminal plasma proteins. The pH of the medium can also determine many important aspects of the structure and function of biological macromolecules, including enzyme activity, and thus can act to determine the behavior of cells. Using these aspects, embodiments of the invention can apply processes and substances in a new way to differentially impact characteristics such as the net charge on the surface of the cell. This can be affected by the pH of the cell's surrounding environment. And the cell can become more positively or negatively charged due to the loss or gain of protons. At or near physiological sperm pH, the net surface charge can be considered as being negative. Further, biological membranes, including sperm, are considered negatively charged in physiological pH. This may be due mainly as a result of the presence of acidic phospholipids; 10 - 20% of the total membrane lipids are anionic ones.

Because the membrane can be exposed to a surrounding aqueous buffer, specific interactions with outer medium components can occur or can be induced by desired processes. A resulting equilibria, in which charged groups of membrane components and solutions ions can be involved, can be affected by different factors and processes leading to a membrane surface charge density variation. The parameter can also be influenced by membrane composition, ionic strength of electrolytes, and solution pH. With this background, it can be understood that viable mammalian sperm can be considered to have a net negative surface charge bound to their plasma membrane. As sperm undergo capacitation followed by the acrosome reaction, it can be considered that their net charge can be reduced and positively charged components can be considered as appearing. Capacitation can thus be considered as characterized by the removal of coating materials from the sperm surface and as the penultimate step in either fertilization resulting in an increased permeability of the plasma membrane to Ca⁺⁺ ions, which allows the sperm to undergo the acrosome reaction or death if fertilization does not occur. Sperm cell's net negative charge increase by capacitation can be demonstrated by selecting sperm based on the negative zeta electrokinetic potential sperm acquire in a specific medium (Chan et al., "A simple zeta method for sperm selection based on membrane charge"). For purposes of understanding how to specifically adapt embodiments of this invention it can be understood that a viable mature human sperm can be understood as having a negative zeta potential of -16 to -20 mV (differential potential between the sperm membrane and its surroundings). Further, this can be understood as decreasing (less negative) upon capacitation and can be understood as becoming more positively charged or at least near zero by capacitation. As further background from which embodiments of the invention can be fine-tuned for specific applications, it should be understood that mammalian sperm are not immediately capable of fertilizing oocytes, rather they must undergo a period of preparation that normally occurs in the female reproductive tract. The changes that occur in sperm can be considered as involving at least two components: an initial sperm membrane alteration that allows the sperm to undergo the second phase, and the fusion of the plasma membrane and outer acrosomal membrane. The first phase can be considered to be the period of capacitation, and the second phase can be considered as the acrosome reaction.

Heparin is a substance that can induce capacitation in sperm leading to fertilization of oocytes. It can be used in embodiments of the invention as one of the ways to achieve inducing a sperm cell sex chromosome related differential effect, to induce capacitation and likely effect a change in the zeta potential of the sperm cells. Surprisingly, this can be a differential effect in X as opposed to Y sperm and can serve as a method to discriminate between the two types of sperm cells. Further, to additionally control the capacitation of the sperm, the pH of the surrounding media can be important. In embodiments of the invention, it can be helpful to introduce the sperm cells to a medium so that the internal pH of the sperm increases from the pH of its baseline ejaculate by some amount, perhaps such as 0.36 pH units. There can also be a time dependence factor where passage of time can cause such desired changes as well. As helpful background to aid in both understanding the invention as well as facilitating available adaptation to specific applications, it may be understood that likely the surface of the sperm during capacitation can lose sialic acid groups as they are incorporated into the sperm membrane. The loss of sialic acid may be responsible for the loss of the net negative charge of live sperm. Further, it appears that human X and Y sperm differ in sialic acid content - Y having less sialic acid than X, and therefore this patent disclosure sets out to demonstrate that sperm bearing X chromosomes can be separated from Y bearing chromosomes based on controlled capacitation of an ejaculate by raising the pH of the ejaculate over time with buffers including heparin, rendering the zeta potential charge of the capacitated sperm neutral to slightly positive and separating the neutral/positive charged sperm with a negatively charged surface or particles yielding a high percentage of X bearing chromosome sperm. Noteworthy is that is it likely that the heparin induced capacitation can cause sialic acid differences in a time period, perhaps such as a six-hour window. The sialic acid differences can contribute to sex selection perhaps based on the differences of sialic acid content and subsequent pH and zeta potential differences of the sperm cells.

As mentioned above, one can control to allow or assure that the differential effect exists when it is desired. A differential effect, such as in but one example, an intrinsically actionable sperm cell sex chromosome related differential effect can be affirmatively supported so that it can be assured to be present when needed. While it may be applied where it exists in other cells, in sperm cells, simply having a stress even can trigger, allow, or induce capacitation and a differential effect. This is true when it is the process of capacitation that is used to cause the differential effect.
Stress, such as changes in environment, freezing, thawing, and others can induce the onset of the capacitation processes. Some embodiments can involve affirmatively establishing the collection of sperm cells in a frozen then thawed state. Once in this state, the process can be timed or otherwise measured to get the desired differential effect. To make the process affirmative and thus both controllable and reliably repeatable and usable such as when following the process of allowing the differential effect to occur, the change can be accomplished and timed or otherwise measured to determine if and when an actionable, appropriate, and perhaps balanced (such as with viability, or cell function non-impairment) differential effect exists for use for ultimately a separation process. In this regard, embodiments of the invention can involve affirmatively establishing a collection of cells, perhaps such as sperm cells, in a timed transition state. The change in environment that is used to cause the desired differential effect can include an appropriate change of buffer; although this can potentially also be considered a chemical change. Embodiments can involve subjecting the collection of sperm cells to a differential change inducing buffer. This differential change inducing buffer can be accomplished through the step of subjecting the collection of sperm cells to a buffer containing an operative amount of a salt, or the step of subjecting the collection of sperm cells to a more basic buffer. Naturally for other cells and even for sperm cells, other types of inducements can be designed within the scope of the invention.

While the differential effect can be allowed to exist in a passive manner without significantly compromising use of any of the cells, it can also be so achieved through the process of inducing the differential effect with an inducer, perhaps such as inducing a sperm cell sex chromosome related differential effect. This step of inducing can cause the differential effect to controllably exist whenever it is desired. The step of timing for the desired effect can achieve a step of controlled difference inducing the effect perhaps such as the sperm cell sex chromosome related differential effect or a differential effect difference control. Inducing the desired effect can be achieved chemically and embodiments of the invention can include a differential effect chemical inducer agent or the step of chemically inducing the sperm cell sex chromosome related differential effect. As mentioned above, one chemical that has been discovered to induce a differential effect in sperm cells based on their sex chromosome so far is heparin. Significantly, it is now disclosed that heparin, a chemical generally considered very safe for fertility control, can be used to safely induce a desired differential effect without significant impairment of the viability of the sperm cells if it is used both properly and controlled appropriately. Not enough or too short a use, and there can be not enough of a differential effect; too much or too long and either the differential can decay or it can go away, and/or the viability of the sperm cells can be unacceptably compromised. Thus, embodiments of the invention can involve subj ecting the collection of sperm cells to heparin and even having an appropriate concentration of heparin and timing or otherwise determining the amount of the change that is optimal for the amount of differential effect and other considerations.

For uses of heparin as a differential effect inducement, subjecting the collection of sperm cells to heparin can include values perhaps including but not limited to: a concentration of 5 ug heparin per ml of buffer, a concentration of 10 ug heparin per ml of buffer, a concentration of 15 ug heparin per ml of buffer, a concentration of 20 ug heparin per ml of buffer, a concentration of 5 ug heparin per ml of buffer for 120 minutes for sperm cells that have not been previously frozen, a concentration of 5 ug heparin per ml of buffer for 180 minutes for sperm cells that have not been previously frozen, a concentration of 5 ug heparin per ml of buffer for from 180 minutes to 240 minutes for sperm cells that have not been previously frozen, a concentration of 10 ug heparin per ml of buffer for 120 minutes for sperm cells that have not been previously frozen, a concentration of 10 ug heparin per ml of buffer for 180 minutes for sperm cells that have not been previously frozen, a concentration of 10 ug heparin per ml of buffer for from 180 minutes to 240 minutes for sperm cells that have not been previously frozen, a concentration of 15 ug heparin per ml of buffer for 120 minutes for sperm cells that have not been previously frozen, a concentration of 15 ug heparin per ml of buffer for 180 minutes for sperm cells that have not been previously frozen, a concentration of 15 ug heparin per ml of buffer for from 180 minutes to 240 minutes for sperm cells that have not been previously frozen, a concentration of 20 ug heparin per ml of buffer for 120 minutes for sperm cells that have not been previously frozen, a concentration of 20 ug heparin per ml of buffer for 180 minutes for sperm cells that have not been previously frozen, a concentration of 20 ug heparin per ml of buffer for from 180 minutes to 240 minutes for sperm cells that have not been previously frozen, a concentration of 5 ug heparin per ml of buffer for 30 minutes for thawed previously frozen sperm cells, a concentration of 5 ug heparin per ml of buffer for 60 minutes for thawed previously frozen sperm cells, a concentration of 5 ug heparin per ml of buffer for from 45 minutes to 60 minutes for thawed previously frozen sperm cells, a concentration of 10 ug heparin per ml of buffer for 30 minutes for thawed previously frozen sperm cells, a concentration of 10 ug heparin per ml of buffer for 60 minutes for thawed previously frozen sperm cells, a concentration of 10 ug heparin per ml of buffer for from 45 minutes to 60 minutes for thawed previously frozen sperm cells, a concentration of 15 ug heparin per ml of buffer for 30 minutes for thawed previously frozen sperm cells, a concentration of 15 ug heparin per ml of buffer for 60 minutes for thawed previously frozen sperm cells, a concentration of 15 ug heparin per ml of buffer for from 30 minutes to 60 minutes for thawed previously frozen sperm cells, a concentration of 20 ug heparin per ml of buffer for 30 minutes for thawed previously frozen sperm cells, a concentration of 20 ug heparin per ml of buffer for about 60 minutes for thawed previously frozen sperm cells, a concentration of 20 ug heparin per ml of buffer for from 30 minutes to 60 minutes for thawed previously frozen sperm cells, until exhibiting an increase of 0.33 pH, until exhibiting an increase of 0.36 pH, until exhibiting an increase of 0.39 pH, until exhibiting an optimal differential effect increase in pH, until exhibiting an optimal cell viability increase in pH, until exhibiting an optimal differential effect increase in pH such as might yield the earliest usable effect, the highest viability, the maximum differential, and until exhibiting an optimal cell viability increase in pH, or any combinations of the above. Caffeine can also serve to induce capacitation and a differential effect. For uses of caffeine as a differential effect inducement, subjecting the collection of sperm cells to caffeine can include designing a system with values perhaps including but not limited to: until exhibiting an increase of 0.33 pH, until exhibiting an increase of 0.36 pH, until exhibiting an increase of 0.39 pH, until exhibiting an optimal differential effect increase in pH such as might yield the earliest usable effect, the highest viability, or the maximum differential, and until exhibiting an optimal cell viability increase in pH, or any combinations of the above. Where a pH altering buffer is used to induce capacitation and thus a differential effect, subjecting the collection of sperm cells to a pH altering buffer (15) can include designing a system with values perhaps including but not limited to: subjecting said collection of sperm cells to a buffer having a pH that increases the environment of said collection of sperm cells by 0.33 pH, subjecting said collection of sperm cells to a buffer having a pH that increases the environment of said collection of sperm cells by 0.36 pH, subjecting said collection of sperm cells to a buffer having a pH that increases the environment of said collection of sperm cells by 0.39 pH, or any combinations of the above.

To illustrate the process in its different aspects and to show its likely efficacy, different examples and test events have been conducted. The first example or test is a sequence designed to illustrate the pH change and likely capacitation state variances in neat sperm over time at room temperature in various buffers with various pH's.

### EXAMPLE 1: Measurement of pH of fresh semen in different buffers over time.

This first example is disclosed to show the pH change in neat sperm over time at room temperature in various buffers with various pH's. As background, it should be understood that the ability to facilitate or induce capacitation is useful in some embodiments such as for magnetic removal of capacitated sperm. Naturally it should be understood that these examples are testing initial options only. As those of ordinary skill in the art should well understand, the pH variances and incubation times set for the sperm samples can be altered to suit particular applications and to create other embodiments that still fall within the scope of the present claims.

As part of this initial test, each of these ejaculates were either kept as neat semen (untouched), resuspended in TRIS 300WS buffer, resuspended in a clear TALP buffer, or resuspended in an Sp-TALP-H (heparin 10 ug/ml) buffer as but initial type of buffers that could be used. Because there was a little over 1 ml per ejaculate, 300 ul of ejaculate was pipetted into each of the four groups. The neat ejaculate was not diluted, whereas the other groups had 700 ul of buffer added to each tube.

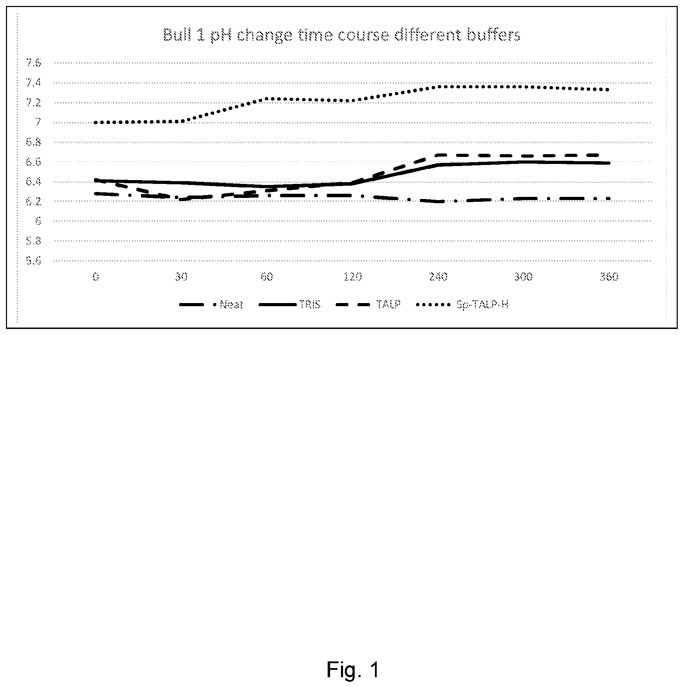

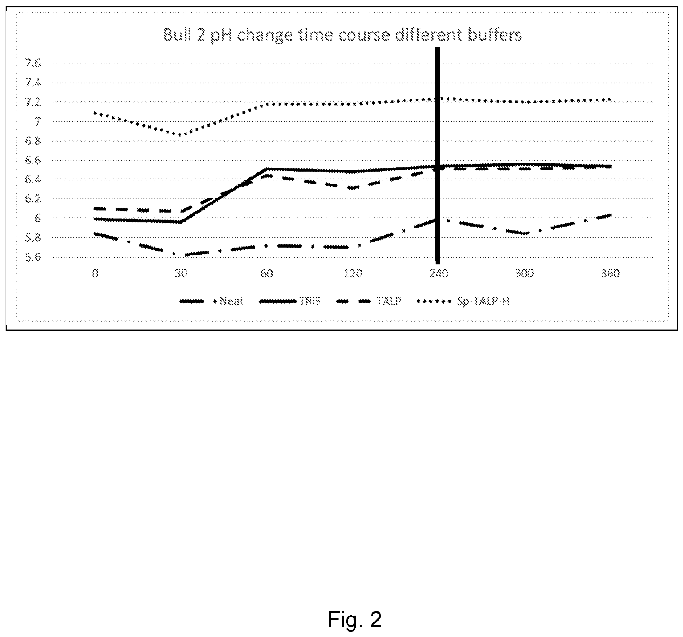

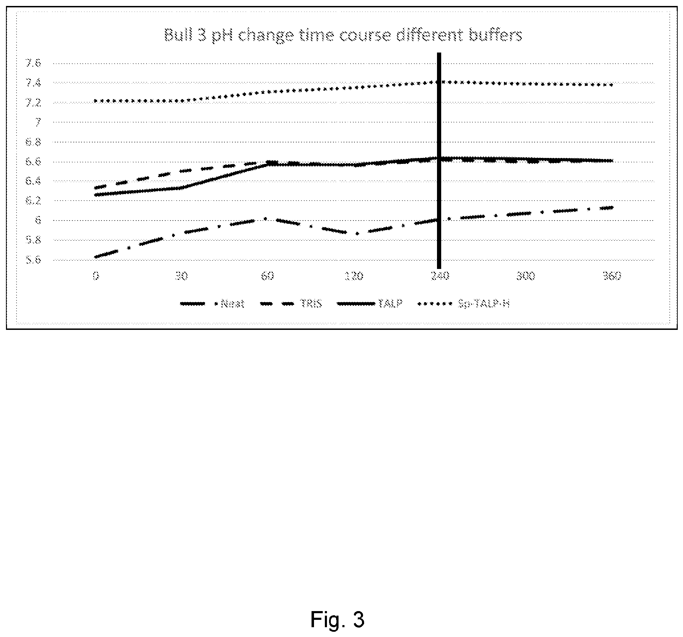

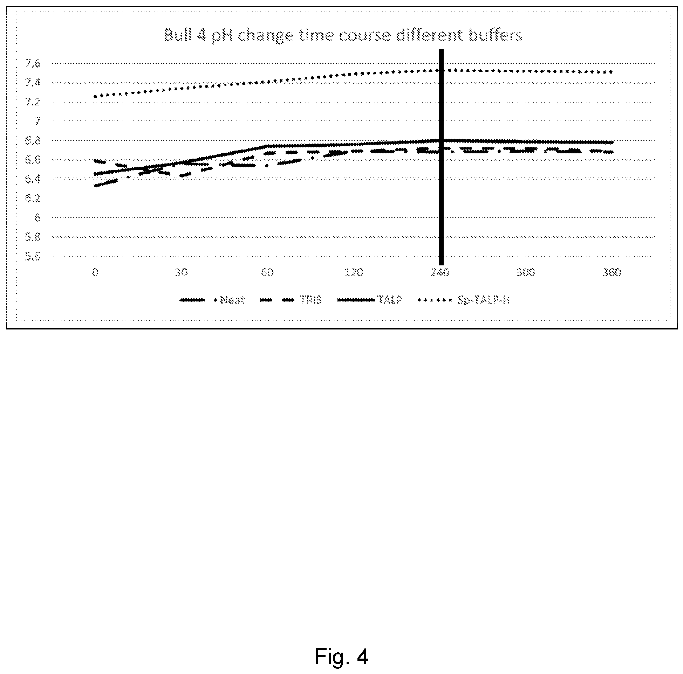

As one example, and as shown for an initial time frame in Figures 1-4, it may be noted that Sperm that was diluted in TRIS WS300 or left to sit at room temperature can maintain a pH range of 6.5 to 6.8 for a long period of time (~24 hrs). Different capacitation media can have different properties and one can likely have more benefit than the other for any specific application. The goal of this experiment was to determine an initial cutoff time period and most effective pH change to alter pH and likely cleave all sialic acid groups off the Y sperm while leaving a number of X sperm available to process. This test assessed a number of variables in this experiment, namely pH over time in various buffers in neat undiluted and diluted ejaculates. An object was to quantify a change of pH in an ejaculate within a given buffer over time. From this it was shown that one step of some embodiments can be to induce capacitation, such as with heparin as in some capacitation buffers, and this step can be used to increase the pH of the overall ejaculate faster than buffers without capacitation elements.

The test protocol involved, any part of which could be used in embodiments of the present invention:
1) One ejaculate from 4 different bulls.
2) Each ejaculate was divided into four groups:
   a) Neat
   b) TRIS 300WS
   c) Clear TALP
   d) Sp-TALP-H (with heparin 10 ug/ml)
3) Because there was a little over 1 ml per ejaculate, 300 ul of ejaculate was pipetted into each of the four groups. The neat ejaculate was not diluted, whereas the other groups had 700 ul of buffer added to each tube.
4) Sampling times - allow at least 2 hrs to pass after ejaculation before processing
   a) Time 0
   b) 30 minutes
   c) 1 hour
   d) 2 hours
   e) 3 hours
   f) 4 hours
   g) 5 hours
   h) 6 hours
5) Take a pH measurement every time period indicated in the tables below. Further, the baseline pH of each buffer is shown in Table 1.

**Table 1. pH measurements of buffers**

| Buffer | Initial pH |
|---|---|
| TRIS 300 WS | 6.92 |
| Clear TALP | 7.01 |
| Sp-TALP-H | 7.44 |

Tables 2 through 5 below show the pH values in each buffer for each bull at a given time period and these are shown in Figures 1-4.

**Table 2: Bull 1 - collected at 8AM sampling occurred at 11:38AM**

| BULL 1 | Neat | TRIS WS 300 | Clear TALP | Sp-TALP-H |
|---|---|---|---|---|
| Time 0 | 6.28 | 6.41 | 6.42 | 7.03 |
| 30 minutes | 6.24 | 6.39 | 6.22 | 7.01 |
| 1 hour | 6.26 | 6.35 | 6.31 | 7.24 |
| 2 hours | 6.26 | 6.38 | 6.39 | 7.22 |
| 3 hours | No Data | No Data | No Data | No Data |
| 4 hours | 6.20 | 6.57 | 6.67 | 7.36 |
| 5 hours | 6.23 | 6.60 | 6.66 | 7.36 |
| 6 hours | 6.23 | 6.59 | 6.67 | 7.33 |

**Table 3: Bull 2 - collected at 8AM sampling occurred at 11:41AM**

| BULL 2 | Neat | TRIS WS 300 | Clear TALP | Sp-TALP-H |
|---|---|---|---|---|
| Time 0 | 5.84 | 5.99 | 6.10 | 7.09 |
| 30 minutes | 5.62 | 5.96 | 6.07 | 6.86 |
| 1 hour | 5.72 | 6.51 | 6.44 | 7.18 |
| 2 hours | 5.70 | 6.48 | 6.31 | 7.18 |
| 3 hours | No Data | No Data | No Data | No Data |
| 4 hours | 5.99 | 6.54 | 6.51 | 7.24 |
| 5 hours | 5.84 | 6.56 | 6.51 | 7.20 |
| 6 hours | 6.03 | 6.53 | 6.53 | 7.23 |

**Table 4: Bull 3 - collected at 8AM sampling occurred at 11:47AM**

| BULL 3 | Neat | TRIS WS 300 | Clear TALP | Sp-TALP-H |
|---|---|---|---|---|
| Time 0 | 5.63 | 6.33 | 6.26 | 7.22 |
| 30 minutes | 5.87 | 6.50 | 6.33 | 7.22 |
| 1 hour | 6.02 | 6.60 | 6.57 | 7.31 |
| 2 hours | 5.86 | 6.56 | 6.57 | 7.35 |
| 3 hours | No Data | No Data | No Data | No Data |
| 4 hours | 6.01 | 6.62 | 6.64 | 7.41 |
| 5 hours | 6.07 | 6.60 | 6.63 | 7.39 |
| 6 hours | 6.13 | 6.61 | 6.61 | 7.38 |

**Table 5: Bull 4 - collected at 8AM sampling occurred at 11:52AM**

| BULL 4 | Neat | TRIS WS 300 | Clear TALP | Sp-TALP-H |
|---|---|---|---|---|
| Time 0 | 6.33 | 6.59 | 6.45 | 7.26 |
| 30 minutes | 6.56 | 6.43 | 6.57 | 7.34 |
| 1 hour | 6.54 | 6.67 | 6.74 | 7.41 |
| 2 hours | 6.69 | 6.69 | 6.76 | 7.49 |
| 3 hours | No Data | No Data | No Data | No Data |
| 4 hours | 6.68 | 6.72 | 6.80 | 7.53 |
| 5 hours | 6.69 | 6.72 | 6.79 | 7.52 |
| 6 hours | 6.68 | 6.69 | 6.78 | 7.51 |

From this data, it can be seen that the sperm TALP buffer with added heparin is one way to increase the pH of the ejaculate by more than a desired amount, perhaps such as 1 pH unit in each ejaculate (Bull 1 = 1.05, Bull 2 = 1.4, Bull 3 = 1.38, Bull 4 = 1.18). This was reached in all ejaculates by the four-hour incubation mark. Naturally other processes and other samples can have varying times and, again, this is shown as one possible embodiment with enough information to permit those skilled in the art to readily assess how to best achieve the desired effects for their specific application within the teaching of the present invention. Noteworthy for this example, it can be seen that after an initial increase in pH, there was no significant increase in pH in any of the ejaculates after the initial time period as shown in Figures 1 through 4. For these initial examples, it was determined that one possible cutoff time sufficient to increase the baseline pH by a desired amount such as 0.36 units pH was between three and four hours in the Sp-TALP-H buffer. From this, it can be understood that buffers with ingredients such as heparin can induce a pH change and presumably capacitation change which can lead to a zeta charge alteration. From this initial test, it appears that the bulls had the most significant increase in pH using the Sp-TALP-H buffer among these four. The concentration of heparin in the Sp-TALP-H buffer was 10 ug/ml. This heparin concentration is also evaluated below for optimal capacitation disparity between X and Y as well as retaining viability of the X population. The increase of at least 0.36 pH units is potentially necessary for the sloughing of sialic acid groups for capacitating sperm. As noted above, this was reached in all ejaculates by the four-hour incubation mark with no increase in pH in any of the ejaculates after that time period. All of the sampling was performed at room temperature.

As mentioned, subtilities such as stress or other things can trigger the desired differential effect. Since when the needed level of the differential effect occurs can be less than immediate for some embodiments, some way of determining or measuring when is the right time to use the cells can be helpful to enable use of the invention. As mentioned, it may be desirable for the process to be timed or otherwise measured to determine when the desired differential effect exists. Not only can the level or rate of transition to the level vary based on the prior conditions of the cells or such, it can be varied based on the planned uses (immediate insemination, overnight storage, freezing, etc.) or next processing events, and it can even be varied based on requirements for viability or cell function. Here, embodiments of the invention can involve determining a usable level of cell differential effect, a differential effect usable level indicator (16), a maximum differential effect difference level indicator, or a differential effect pH indicator, and affirmatively effecting that level of cell differential effect for the collection of cells, or for the sperm cell applications. Such can include determining a usable level of sperm cell sex chromosome related differential effect, and affirmatively effecting that level of sperm cell sex chromosome related differential effect for the collection of sperm cells. Determinations can be made based on a predetermined amount of time after/since a known activity (thawing, change of buffers, etc.), or they can be made based upon direct measurements or some combination of these, of course. However accomplished, embodiments can include the step of determining something, perhaps such as a maximum difference level or maximum distinction between the two types of cells, perhaps the maximum difference level of sperm cell sex chromosome related differential effect, or determining a usable pH indicated level of cell or sperm cell sex chromosome related differential effect. As explained in detail above, an increase in pH above some determined amount can be potentially necessary to enable implementation of some embodiments of the invention. Variation in pH increase determinations can include: determining a pH increase for the environment of said collection of sperm cells of determining a pH increase for the environment of said collection of sperm cells of 0.33 pH, determining a pH increase for the environment of said collection of sperm cells of 0.36 pH, and determining a pH increase for the environment of said collection of sperm cells of 0.39 pH. Variation in timed differential effect determinations can include generally timing a cellular process differential transition effect for the collection of sperm cells or options including but not limited to terminating the cellular process differential transition effect immediately, terminating the cellular process differential transition effect at 90 minutes, terminating the cellular process differential transition effect at 120 minutes, terminating the cellular process differential transition effect at 150 minutes, terminating the cellular process differential transition effect at up to 150 minutes, utilizing frozen-thawed sperm cells after having been thawed for 4 hours, utilizing frozen-thawed sperm cells after having been thawed for 6 hours, utilizing frozen-thawed sperm cells after having been thawed for 8 hours, utilizing frozen-thawed sperm cells after having been thawed for 12 hours, and utilizing frozen-thawed sperm cells after having been thawed for overnight.

Returning to the example, the TRIS and clear TALP buffers maintained a pH of around 6.8 in all of the ejaculates throughout the duration of the study and therefore advantages of these for specific processes would need to be weighed against the potential that such may cause slower capacitation and thus having impacts on the separation of X from Y in a timely manner. These buffers might also be a good buffer to transfer sperm into once the capacitation reaction is completed perhaps if such were desired to be quenched as may often be desired. Quenching the induced sperm cell sex chromosome related differential effect or quenching the transition can be useful to achieving a desired cell viability. This can be achieved by a differential effect pause element (11) or pausing the effect at a desired phase such as by the step of pausing an intrinsically actionable sperm cell sex chromosome related differential effect. For example, it is possible that when the collection of sperm cells initially exists in seminal plasma, it may be desired to remove the seminal plasma from the collection of sperm cells so as to have a seminal plasma-less collection to allow their transition to occur more appropriately or perhaps even relatively immediately. In this embodiment, the step of pausing the transition can even be accomplished by the act of resubjecting the collection of sperm cells to a seminal plasma mixture (to make a seminal plasma included collection) or to BSA (bovine serum albumin) for a BSA included collection or some other appropriate substance. The aspect of pausing the differential effect can be momentary or can be complete as when pausing occurs as a stop to any further progress of the effect or transition.

All these can achieve the step of quenching capacitation of the collection of sperm cells or the element of providing a quencher (17) and embodiments can achieve subjecting the collection of sperm cells to a mixture containing substances selected from a group consisting of: seminal plasma, or BSA.
In some embodiments of the present invention, sperm might be capacitated in a titrated manner perhaps to exploit differences in amino acids that may be cleaved during the capacitation process. This might reveal differences in net zeta potential charge between X bearing sperm and Y bearing sperm as well. For example, sperm could be pre-incubated such as with Sp-TALP for perhaps 6 hours and 24 hours. Maturation in media such as for 24 hours, washing perhaps multiple times such as in Talp-Hepes, and in IVF-Talp and then transferal into 500 µl of IVF medium supplemented with 20 µg/ml of heparin could also be used with consideration of the specific applications as appropriate. Sperm could also be pre-incubated such as in Sp-TALP (perhaps with no heparin) such as at 39C with 5% CO₂ for 0, 6, and 24 hours. At some point, perhaps such as the fifteen-hour mark, sperm could be centrifuged and media replaced.
Some period of incubation in heparin, such as a four-hour incubation with 10 ug/ml of heparin, or perhaps a four-hour incubation in heparin stock preparation of 170 units/mg dissolved in saline, with 10 ug/ml stock heparin, could be used to cause capacitation of at least bovine sperm. In addition, it is possible that shorter period of incubation could be used, for example prepurified X and Y semen were incubated in Sp-TALP-H with a heparin concentration of 10 ug/ml for three-hours at room temperature and this process showed that 50.82% of Y sperm underwent capacitation and/or death in this capacitation buffer, while only 16.88% of X sperm went through capacitation and/or death. Interestingly, the pH in both of these samples was at 7.5, while the starting pH of both samples was 6.8. Thus, as a person of ordinary skill would well understand and be able to determine for specific applications how to best apply the invention to achieve inducement of capacitation in varied manners.

As described in more detail below, ultimately, in embodiments, processes can include mixing and even incubating the collection of sperm cells with associatable particles. Particles mixed with cells can then be used as part of the separation process or modality. Here, embodiments can involve combining cells with associatable particles, and establishing a fluid combination of the associatable particles and the cells. For sperm cell applications, there can be sperm cell associatable particles with the collection of sperm cells to establish a fluid combination of sperm cell associatable particles and sperm cells. Once this combination is established, system can achieve associating a desired portion of the collection of cells or sperm cells with at least some of said associatable particles in the fluid combination. Then the step of separating at least some of the desired types of cells, perhaps the X-bearing sperm cells and Y-bearing sperm cells, can be achieved through action of the associatable particles in the fluid combination.

Having generally described the aspect of inducing capacitation to cause the desired effect, more details are presented in the following examples. Again, it should be understood that, although examples involve initial steps and even sperm cells as the initial cell item, the steps and this selection of cell or application is not intended to limit the scope of the present invention as indeed other type of cells may indeed prove valuable in applications of the general teaching of the present invention.

### EXAMPLE 2: Measurement of pH of pre-purified X and Y semen and conventional semen without seminal plasma in buffers over time.

This second example is disclosed to compare the pH change in X bearing and Y bearing sperm over time. The same buffers used in the first example were used (TRIS, Clear TALP, and the Sp- TALP-H). It shows the surprising and unanticipated differential that the two types of sperm achieve and lays a foundation for the invention whereby a difference that can be exploited and acted upon to achieve a sex related bulk separation can be created in some embodiments. As this example shows, Sp-TALP-H increased the percent capacitated Y sperm more than that of the X sperm with a heparin addition of 10 ug/ml. Naturally, other ranges of heparin concentration to induce capacitation as well as longer incubation periods to induce capacitation can be developed for each application. One goal of this experiment was to determine the cutoff time period and most effective heparin concentration to cleave all sialic acid groups off the Y sperm.

In order to determine the pH rate or amount of change in X chromosome bearing sperm versus Y chromosome bearing sperm, pre-purified X and Y semen were incubated in the aforementioned buffers over time and compared to conventional semen pH rate change. These samples were frozen thawed pre-purified X and Y sperm as was separately obtained. Additionally, capacitation rates were measured for each population with the pH increase to see how they differed from one another. Two million cells for each aliquot from each group were washed with the buffer in which they were to be incubated. Each sperm pellet was resuspended in 4 ml of the designated buffer. For each population the pH and capacitation rate or amount was measured at time 0, 30 minutes, 1 hour, 2 hours and 3 hours after incubation with the designated buffer as shown in tables 6 through 8. An object of this example is to quantify the change of pH in a given buffer from pre-purified sperm measured in different buffers over time, and also to quantify the capacitated sperm by flow cytometry correlating to the pH change and time period.

This example shows that inducing capacitation with heparin as in some capacitation buffers can increase the pH of this overall ejaculate faster than others. It also shows that this process can lead to a larger population of dead cells than seen in typical processing buffers. It supports the possibility that Y sperm may contain less sialic acid groups; therefore, the heparin inducing capacitation buffer may cause the Y sperm to capacitate more rapidly than X sperm.
This test protocol involved, any part of which could be used in embodiments of the present invention:
1) Three groups from each semen type:
   a) X (each tube contains 2 mls for a total of 4 x 10⁶ cells)
      i. TRIS WS 300 (2 x 10⁶ sperm/ml)
      ii. Clear staining TALP (2 x 10⁶ sperm/ml)
      iii. SP-TALP-H (2 x 10⁶ sperm/ml)
   b) Y (each tube contains 2 mls for a total of 4 x 10⁶ cells)
      i. TRIS WS 300 (2 x 10⁶ sperm/ml)
      ii. Clear staining TALP (2 x 10⁶ sperm/ml)
      iii. SP-TALP-H (2 x 10⁶ sperm/ml)
   c) Mixed (20 x 10⁶ cells divided into three groups)
      i. TRIS WS 300 (6.7 x 10⁶ sperm/ml)
      ii. Clear staining TALP (6.7 x 10⁶ sperm/ml)
      iii. SP-TALP-H (6.7 x 10⁶ sperm/ml)
2) Each sperm pellet was resuspended in 4 ml of the buffer they were indicated.
3) Sampling times - measure pH and fluorescence with flow cytometry
   a) Time 0
   b) 30 minutes
   c) 1 hour
   d) 2 hours
   e) 3 hours

Measurements of pH were made every time period indicated in the table below.

**Table 6. X chromosome bearing semen.**

| X semen | TRIS WS 300 | % Capacitated | Clear TALP | % Capacitated | Sp-TALP-H | % Capacitated |
|---|---|---|---|---|---|---|
| Time 0 | 6.67 | 1.9 | 6.77 | 4.97 | 7.47 | 16.11 |
| 1 hour | 6.73 | 4.12 | 6.81 | 3.54 | 7.54 | 16.6 |
| 2 hours | 6.74 | 3.9 | 6.83 | 3.92 | 7.56 | 16.17 |
| 3 hours | 6.74 | 4.09 | 6.82 | 4.33 | 7.58 | 16.88 |

**Table 7. Y chromosome bearing semen.**

| Y semen | TRIS WS 300 | % Capacitated | Clear TALP | % Capacitated | Sp-TALP-H | % Capacitated |
|---|---|---|---|---|---|---|
| Time 0 | 6.69 | 54.35 | 6.73 | 15.81 | 7.51 | 46.11 |
| 1 hour | 6.72 | 47.03 | 6.78 | 19.32 | 7.53 | 49.27 |
| 2 hours | 6.72 | 45.38 | 6.77 | 18.31 | 7.54 | 45.3 |
| 3 hours | 6.75 | 47.22 | 6.76 | 18.52 | 7.48 | 50.82 |

**Table 8. Conventional semen (mixed X and Y sperm).**

| Conventional | TRIS WS 300 | % Capacitated | Clear TALP | % Capacitated | Sp-TALP-H | % Capacitated |
|---|---|---|---|---|---|---|
| Time 0 | 6.69 | 28.83 | 6.77 | 35.1 | 7.50 | 30.79 |
| 1 hour | 6.72 | 18.93 | 6.77 | 31.9 | 7.57 | 28.53 |
| 2 hours | 6.71 | 18.90 | 6.83 | 31.29 | 7.59 | 27.83 |
| 3 hours | 6.69 | 15 | 6.81 | 32.92 | 7.58 | 28.88 |

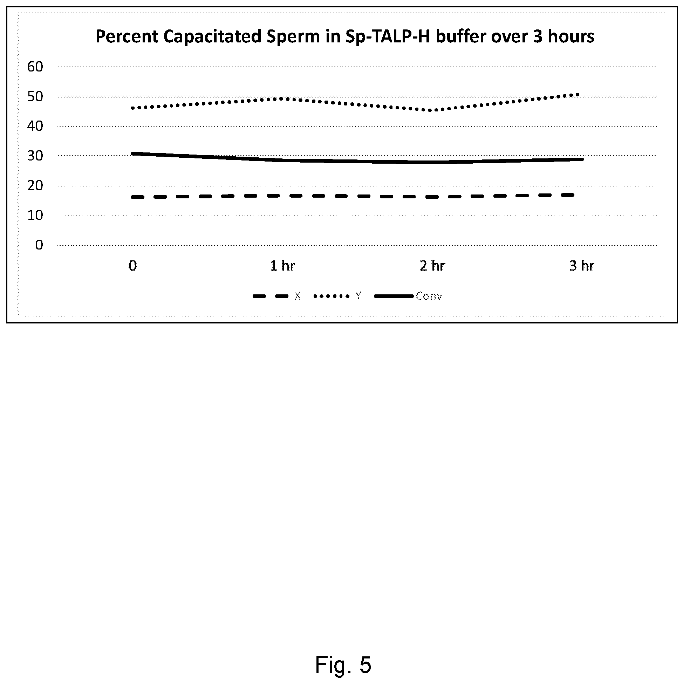

As the above data shows, surprisingly, there is a stark difference in pH and likely percent capacitation over time for X bearing sperm versus Y bearing sperm. This is shown in Figure 5. As now disclosed, this difference in capacitation rate or amount can be used to separate the Y sperm from the X sperm. This is perhaps achieved by exploiting the overall difference in electrochemical charge on the surface of the Y sperm versus the X sperm after capacitation reaction has begun and this difference appears to be even more acute. Indeed, as shown below it may be the basis for which a total separation with complete purity can be achieved. The slower capacitation rate or total percentage of the X bearing sperm perhaps can be considered as retaining the net negative charge on the surface of the X sperm while the Y sperm lose their net negative charge perhaps by going through capacitation faster; thereby rendering the Y sperm neutral or slightly positive.

From this example, it can be seen that more Y sperm appear to be capacitated (50%) post thaw than either X sperm (17%) or conventional semen in all buffers after 3 hours. In addition, it can be seen that there is a significantly higher percent of capacitation in Y sperm than in X sperm when incubated in the Sp-TALP-H buffer. In addition, an interesting trend noticed on the dot plots generated from the flow cytometer was a change in the peaks of absolute dead sperm to more dying sperm. From this, it can be understood that the sperm might be sticking to the wall of the tube when finally dead over incubation periods. This might be utilized to refine the process for some applications. Noteworthy is that the pH may not alter that much from the buffer the frozen thawed semen in which the sperm are resuspended. This may be due to the sperm having no seminal fluid and/or buffer to alter the pH given that they are washed prior to resuspension. Further, it may be desirable to remove seminal plasma from neat ejaculates in order to control the capacitation reaction more precisely.

### EXAMPLE 3: Measurement of pre-purified Y semen capacitation via pH in differing concentrations of heparin in Sp-TALP-H buffer over time.

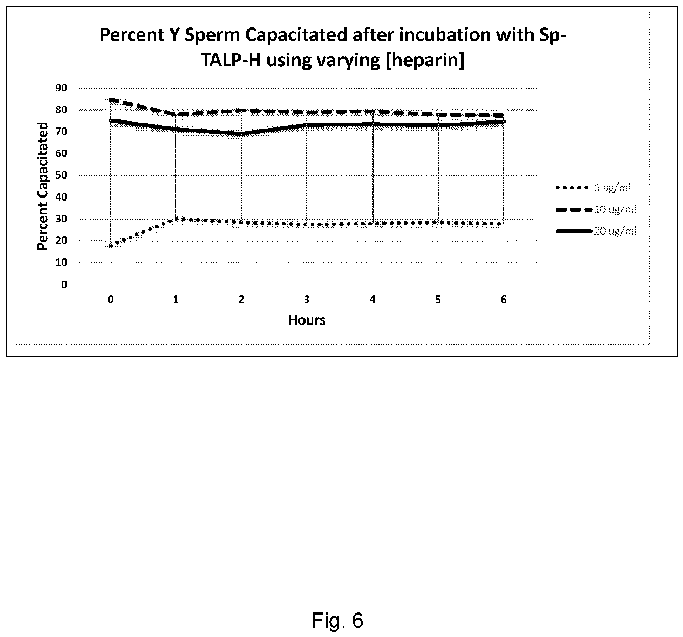

This third example is disclosed to compare the pH change in Y bearing sperm over time for differing concentrations of heparin. There are different medias that contain differing concentrations of heparin to induce capacitation. Varying concentrations of heparin were added to the Sp-TALP-H buffer and incubated with Y to determine higher or lower capacitation rates or amounts in the same amount of time. Three groups were analyzed, 4 million Y sperm each in: 1) Sp-TALP-H (5 ug/ml heparin), 2) Sp-TALP-H (10 ug/ml heparin), 3) SP-TALP-H (20 ug/ml heparin). The cells were then washed with the TRIS WS300 buffer. Each sperm pellet was resuspended in 4 ml of the buffer they were indicated. The pH and capacitation rate were measured over 6 hours as shown in Table 9 and Figure 6.

As shown, it can be seen that by inducing capacitation such as with heparin or some otherwise appropriate capacitation buffer, the pH of the overall ejaculate can be increased faster than merely by the passage of time. Such processes can also lead to a larger population of dead cells than seen in typical processing buffers. As mentioned above, it may be that Y sperm perhaps contain less sialic acid groups; therefore, the heparin inducing capacitation buffer may cause the Y sperm to have capacitated populations more rapidly than X sperm. It is also shown that the number of Y sperm capacitated may not be proportional to the concentration of heparin added to the capacitation buffer within the ranges of interest.

This test protocol involved the following processes, any part of which could be used in embodiments of the present invention:
1) Three groups:
   a) Y (each tube contains 2 mls for a total of 4 x 10⁶ cells)
      i. Sp-TALP-H (5 ug/ml heparin)
      ii. Sp-TALP-H (10 ug/ml heparin)
      iii. SP-TALP-H (20 ug/ml heparin)
2) Each straw was thawed and placed into a 50 ml tube. The cells were then washed with the TRIS WS300 buffer. Each sperm pellet was resuspended in 4 mls of the buffer indicated.
3) Sampling times - measure pH and measure fluorescence with flow cytometry
   a) Time 0
   b) 1 hour
   c) 2 hours
   d) 3 hours
   e) 4 hours
   f) 5 hours
   g) 6 hours

A fluorescence measurement was done by taking a 600 ul aliquot from each stock solution and adding 5 ul of propidium iodide.

**Table 9. Capacitation rate of Y semen incubated with varying concentrations of heparin.**

| Y semen | Sp-TALP-H 5ug heparin % Capacitated | Sp-TALP-H 10ug heparin % Capacitated | Sp-TALP-H 20ug heparin % Capacitated |
|---|---|---|---|
| Time 0 | 33.73 | 84.75 | 75.06 |
| 1 hour | 30.12 | 77.76 | 71.06 |
| 2 hours | 28.54 | 79.64 | 68.98 |
| 3 hours | 27.34 | 78.70 | 73.02 |
| 4 hours | 27.94 | 79.18 | 73.44 |
| 5 hours | 28.50 | 77.78 | 72.80 |
| 6 hours | 27.66 | 77.36 | 74.52 |

As can be seen, the concentration difference of heparin between 10 ug/ml and 20 ug/ml did not cause a remarkable difference in capacitation over time for these samples and these species; however, the increase of heparin from 5 ug/ml to 10 ug/ml and 20 ug/ml did make a significant difference in the percent capacitated. There is also some possibility that optimization of other buffers and the combinations of buffers may be applied in developing processes for specific applications. For example, having too large an amount of other buffer, perhaps such as TRIS buffer, remaining in the sperm might act to stabilize the pH, thus preventing or slowing capacitation. As can also be seen, in these initial examples, the amount of time does not appear to alter the percent capacitated. Considerations of a frozen thawed, washed effect might also be utilized. For example, it may be considered that the sperm may already be damaged from the sorting, freezing, thawing process and may be more susceptible to capacitation initially than Y sperm from a neat ejaculate. As those of ordinary skill in this art would well understand, this data can be used to optimize the invention for specific applications.

### EXAMPLE 4: Zeta Potential Measurement of Carboxy Terminated Iron Oxide Particles in Sp-TALP-H buffer with either 10 ug/ml of heparin or 20 ug/ml of heparin.

In various embodiments of the present invention, some type of separation modality can be used. These modalities can include, but are not limited to, the use of items in solution or even surfaces. An example of one type of item that can be used in solution is magnetic nanoparticles. Magnetic nanoparticles can also be associationally active with the cells to affect a separation of those cells. To the extent that the cells differentially achieve zeta potentials, magnetic nanoparticles can be used because they have a net negative zeta potential without further surface manipulation. This property can be used to bind sperm perhaps as they begin to capacitate and die. As mentioned, because sperm as they begin to capacitate have an increase in intracellular pH which appears to cause the membrane of the capacitated and/or dead sperm to lose their net negative zeta potential, the shift towards a neutral (zero) or more positive zeta potential can be used with magnetic nanoparticles. This can allow the negatively charged magnetic particles to bind specifically to those sperm that are becoming less negative and more neutral or positively charged. In general, processes can be used to titrate such differences in X bearing and Y bearing sperm.

The fourth example shows these features comparing effects for two different concentrations of heparin in the Sp-TALP buffer. It shows the zeta potential of iron nanoparticles in Sp-TALP-H in 10 ug/ml of heparin and the zeta potential of iron nanoparticles in Sp-TALP-H in 20 ug/ml of heparin. And this shows that both serve as a potential collection and/or separation mechanism. The particles demonstrate the appropriate properties desired to provide an embodiment that can be used to remove the Y population from the X bearing sperm. As mentioned, these sperm can be induced to display differential properties, potentially based on a difference in capacitation rate. This difference can be the cell's zeta charge, and in this example, the magnetic particles used to collect the capacitated sperm have an opposite zeta potential charge of one of the sperm, the collected capacitated sperm. This creates a "biological salt" to achieve separation.

As background, it may be noted that a particle with a negatively induced charge such as the carboxyl modified silane surface coating on the magnetic nanoparticles can act to bind to the membrane of the sperm through the apparent electrical charge interaction known as zeta potential. In one embodiment the fact that the material can spontaneously acquire a surface electrical charge when brought into contact with a polar medium (i.e. water) is used. Generally, an interface in deionized water is negatively charged, but there are materials that can be positively charged. An ionization of surface groups whereby a surface gains an electrical charge is observed with all metal oxide surfaces (M-OH) as well as materials that contain carboxyl and/or amino groups. This latter category includes proteins, ionic polymers, and polyelectrolytes. The ionization and/or dissociation of these groups (degree of charge development) and the net molecular charge (and thus the sign, either positive or negative) can depend strongly on the pH of the dispersion media and this can be used to advantage in embodiments of the present invention. In an embodiment of the invention, silane containing carboxyl groups may be used to perhaps result in negative zeta potential magnetic particles in capacitation buffer containing heparin perhaps such as with a pH of 7.5. These may result in the binding of capacitated and dead cells in a composition of mixed sex sample of cells. Through such processes, the Y cells may became more positively charged than the X cells in the capacitation buffer over time allowing for their removal and purification of the X sperm population.

For example, in embodiments of the present invention exploiting the zeta charge of the cells, separation can be effected with any type of magnetically identifying separating apparatus, including but not limited to devices incorporating columns, such as the Miltenyi magnetic-activated cell sorting (MACS) products, devices using simple magnetic fields applied to test tubes or containers, or other high throughput magnetic devices. As those of ordinary skill in the art would realize, any other substrate or device having a charged surface whereby the capacitated sperm may bind can also be used, including but not limited to silanes, glass surfaces, dextrans, sephacryl beads.

In considering magnetic nanoparticles for such a process, it can be understood that with the minimal difference in capacitation rate of the Y sperm in the presence of heparin at a concentration of 10 ug/ml or 20 ug/ml in Sp-TALP-H buffer, the zeta potential of iron oxide particles resuspended in either of these buffers can be used. Further, it can be understood that there is not significant zeta potential measurement difference for such particles between these two buffers. Particles that were carboxy terminated at an iron concentration such as 20 mg/ml with a mean diameter such as 0.67 microns can be collected magnetically. A portion, perhaps such as 2 mg can then be resuspended in 1 ml of an appropriate buffer. In this example, two such buffers were used, Sp-TALP-H, heparin 10 ug/ml and Sp-TALP-H, heparin 20 ug/ml. These were measured on a zeta sizer.

The zeta potential of these carboxyl functionalized iron oxide nanoparticles resuspended in the Sp-TALP-H buffer at either concentration of heparin showed no remarkable difference. They both have strong zeta signals which means they will not aggregate in solution but will flocculate when added to what is likely capacitating sperm to create a biological salt. Noteworthy is that the particles resuspended in Sp-TALP-H with 10 ug/ml of heparin had a mV reading of -21.1 and particles resuspended in Sp-TALP-H with 20 ug/ml of heparin had a mV reading of -23.1 so both can likely be used appropriately for these purposes.

As those of ordinary skill in the art would understand, zeta potential is described as including colloidal particles dispersed in a solution which are electrically charged due to their ionic characteristics and dipolar attributes. Each particle that is dispersed in solution can be considered as surrounded by oppositely charged ions called the fixed layer. Outside the fixed layer, there is likely varying compositions of ions of opposite polarities - forming a cloud like area. This areais called the diffuse double layer, and the whole area is often considered electrically neutral. When a voltage is applied to the dispersed particle solution, the particles are attracted to the electrode of the opposite polarity, accompanied by the fixed layer and importantly only a part of the diffuse double layer, or internal side of the "sliding surface". This aspect suggests that some incubation to achieve the desired charge may be appropriate in particular applications. The zeta potential can therefore be considered to be the electric potential of this inner area including the conceptual "sliding surface". As this electric potential approaches zero, particles may aggregate over a varying amount of time.

While the above particular application is explained in the context of sperm cells and in the context of zeta charge differentials, cleaving, or other surface changes, it should be understood that the disclosure is founded on principles that can be altered and designed for various cells, combinations with various substances, and including various separation modalities.

For example, the aspect of associating a desired portion of a collection of cells with a substance is can encompass a variety of aspects within its requirement of associating. In can, of course encompass associating a desired portion of a collection of cells with a substance, associating a desired portion of cells with cell associatable particles, associating a desired portion of a collection of sperm cells with at least some sperm cell associatable particles, differentially associating a specific chromosome bearing type of sperm cell with said sperm cell associatable particles, as well as associating a desired portion of a collection of sperm cells with sperm cell associatable particles in a fluid combination, to name just a few options. The association mechanisms can vary as well to suit available substances, or desired substances. The association can occur due to surface effect, charge effects, chemistry, organic chemistry, or other means. It can involve electrostatically associating a desired portion of a collection of cells or sperm cells with associatable substances, particles, or surfaces, as well as chemically associating a desired portion of a collection of cells or sperm cells with substances, particles, or surfaces. Naturally the associations themselves can be differential in that they exist for one type of cell and not another and in this manner, embodiments can involve differentially associating with the cells in the collection of cells. Charges giving rise to the association can be based upon sialic acid group content and the step of separating, such as for at least some of the X-bearing sperm cells and Y-bearing sperm cells, can be based on a sialic acid group content of the cells or sperm cells. Associations involving combinations with antibodies (perhaps bound to particles or such) can be designed into systems as well. Importantly, while for one embodiment it is explained that likely X-bearing sperm have a larger surface area than Y-bearing sperm and therefore X-bearing sperm may have more sialic acid groups which contribute to their net negative charge prior to capacitation and death, this is not to be a limitation as to how the disclosed method can be applied. Similarly, the explanation that as the sperm capacitate and lose their net negative charge they become positively charged in certain buffers and situations, and the Y-bearing sperm become positively charged faster than the X-bearing sperm and therefore can be collected at a time interval before X-bearing sperm become or mostly become positively charged as well are not limiting. Even embodiments focusing on steps such as having a pH change (for sperm likely at least 0.36 pH units from the initial starting pH in neat semen), there being sialic acid cleavage and/or binding of groups (other carbohydrates) that contribute net negative charge on non-capacitated living sperm, and even there being particles with an opposite charge (perhaps negative) that have an ability to bind such as to positively charged sperm as they capacitate are to be understood as only a type of embodiment that can be accomplished. Factors or variations that can be implemented in a practical application of the invention can include: a removal of sialic acid groups (as in capacitation), removal that can be accomplished with or without seminal plasma in solution, a removal that can occur naturally with time (for sperm perhaps up to 24 hours just sitting in buffer or seminal plasma), a activities that can occur at room temperature or perhaps somewhat lower, activities that can occur in a water bath up to 39C or perhaps higher, activities that can occur just from thawing frozen cells (for example for sperm freezing does have an effect on the sperm membrane and Y-bearing sperm appear more susceptible to a faster capacitation rate post thaw potentially due to that damage), activities that can involve first freezing and then thawing to separate X-bearing and Y-bearing sperm based on capacitation rate and loss of the negatively bearing sialic acid or carbohydrate groups or such, activities where the changes such as capacitation can be induced or perhaps just amplified by certain buffers including but not limited to buffer with additives such as salts, more basic buffers (increasing the pH), Heparin additives, caffeine additives, including particles that can bind to sialic acid groups or other negatively bearing groups on the sperm surface, particles that include coating such as SNA ligands, antibodies, particles that have the opposite electrical charge (negative zeta) of capacitated sperm (positive charge).

### EXAMPLE 5: Removal of capacitated and semen with iron oxide particles in escalating concentrations in Sp-TALP-H buffer with 20 ug/ml of heparin.

Of course, the ultimate goal of embodiments of the present invention is to effectively separate X and Y bearing sperm. The fifth example shows processes to achieve such a separation. In this example, Y semen was incubated with Sp-TALP-H buffer with 20 ug/ml heparin for six hours at room temperature to capacitate the sperm. An increasing amount of particles was added to the aliquots to show an increase in particles at a level that removed all capacitated and dead sperm. As background and as mentioned above, healthy viable sperm can have a net negative zeta potential. As they begin to capacitate, they can lose this negative charge or can be considered as more prone to becoming zeta resultant slightly positive and can become neutral and/or slightly positive. With negatively charged particles in a buffer which may induce capacitation of Y sperm more rapidly or to a greater degree than X sperm, the potentially more capacitated Y sperm may be attracted to the negatively charged particles or surface with a greater percentage and allow a purified X population to remain in the supernatant (nonmagnetic fraction).

This test protocol involved the following processes, any part of which could be used in embodiments of the present invention:
1) One straw of Y semen was thawed and washed to address extender present by adding 4 ml of Sp-TALP-H buffer and centrifuging.
2) The semen was resuspended in 6 ml of Sp-TALP-H buffer with 20 ug/ml heparin and BSA.
3) One ml of each resuspended cell pellet was then pipetted into six different 50 ml conical tubes.
   a) Unlabeled - control with PI
   b) 0.3 mg particles
   c) 0.6 mg particles
   d) 1.2 mg of particles
   e) 2 mg of particles
   f) 4 mg of particles
4) The appropriate amount of particles to each aliquot so that the total of the particles added equals 100 ul of the total volume.
5) Aliquots were incubated for 20 minutes.
6) After the 20-minute incubation period perhaps at room temperature (this might be done at an elevated temperature, or at some other appropriate temperature) expired for those samples containing particles, they were placed in front of a magnet for three minutes. The nonmagnetic fraction was then aspirated out of the tube and placed into a 12 x 75 mm FACS tube.
7) From each sample after each magnetic separation was complete, aliquots were analyzed by flow cytometry for percent capacitated prior to particle treatment as well as the percent capacitated after particle treatment by adding 10 ul of propidium iodide into the sample and looking for uptake of fluorescence.

In the above, the pre-incubation of Y sperm and X sperm was performed in the Sp-TALP-H buffer with 20 ug/ml for 3 hours. Semen was resuspended in 4 ml of Sp-TALP-Hbuffer with 20 ug/ml heparin and BSA, so that the final cell concentration was 160 million cells/ml. Eight milligrams of magnetic particles were removed from dH₂O, magnetically collected, and resuspended in 1 ml of Sp-TALP-H buffer with 20 ug/ml of heparin. The semen and particles were incubated for 20 minutes at room temperature. After the 20-minute incubation period had expired, for those samples containing particles - they were placed in front of a magnet to potentially induce a desired net zeta charge and also to allow migration for three minutes. The results of the aliquots analyzed by flow cytometry for the percent capacitated cell removal after particle treatment are presented in Table 10.

**Table 10: Percent of cells removed after capacitation with magnetic nanoparticles.**

| Sample ID - Y sperm | Percent Cells in Supernatant | Sample ID - X sperm | Percent Cells in Supernatant |
|---|---|---|---|
| Control | 100 | Control | 100 |
| 1.2 mg particles | 0 | 1.2 mg particles | 83 |
| 2 mg particles | 0 | 2 mg particles | 80 |
| 4 mg particles | 0 | 4 mg particles | 72 |

As can be seen from Table 10, there is a noticeable and remarkable difference in the numbers of cells in the supernatant. In each of these tests, the supernatants contained diametrically opposite results for the Y versus the X sperm. No Y sperm cells remained in the supernatant, whereas virtually all of the X sperm cells (likely less either an insignificant portion or perhaps only the dead or dying cells) remained in the supernatant. Likely this is due to the differing capacitation rates or total percentage of capacitated cells for Y sperm versus X sperm in the capacitation buffer with 20 ug/ml of heparin. This result was not anticipated, nor an obvious outcome. Further, when these samples are mixed, it is expected that the identical results will combine, namely, that the result will be the sum or 83/200, 80/200, 72/200 each presented as pure X sperm results with likely only some significant portion of the X sperm remaining unassociated with the magnetic nanoparticles and therefore remaining in the post magnetic separation supernatant. This will provide a truly bulk sex related separation process that can be optimized and adapted to the various species, applications, and situations according to the teachings of the present invention.

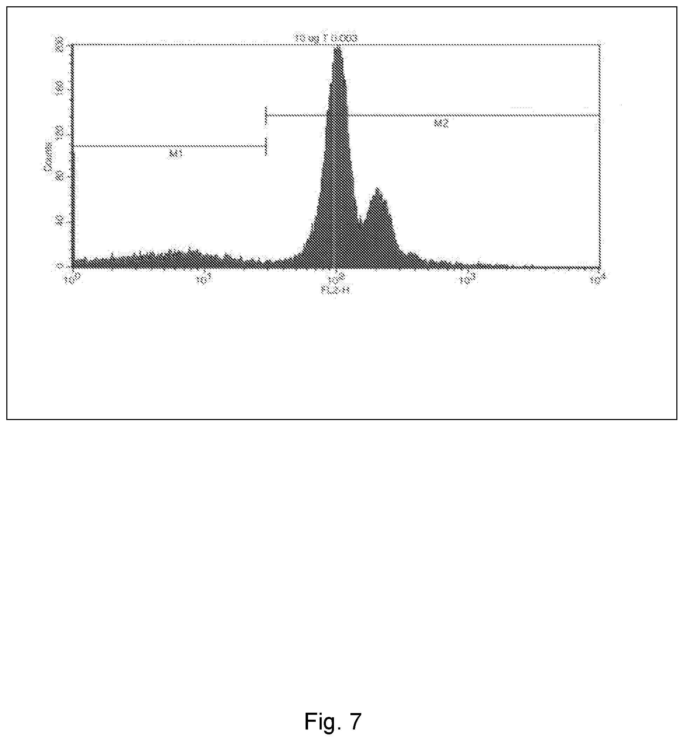

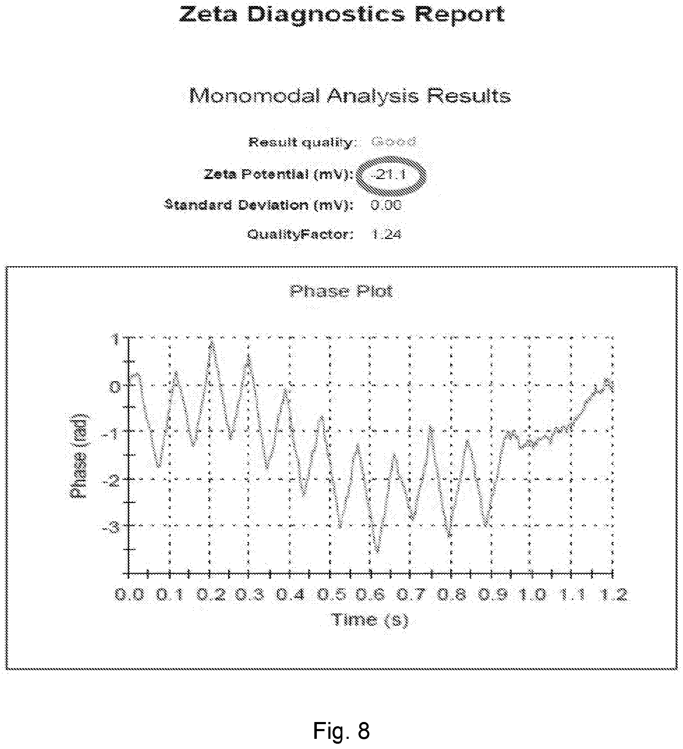

In addition, as can be seen, adding the particles at various concentrations may result in no improvement in the percent of capacitated Y sperm removed (Figures 7 and 8), but may result in an increase in the number of X sperm removed. A higher concentration of magnetic particles in this example may also not be necessary to remove all capacitated cells; while leaving a higher concentration of X cells available for processing and freezing for future artificial insemination use. Other substrates containing a negative zeta potential charge or surfaces containing an external electric charge potential can be used for the removal of these pretreated cells. To effect a separation of the two different types of cells, different mechanisms are possible. In contrast to the non-enabled techniques proposed whereby the different sexed sperm ought to simply swim apart or turn into some type of a 2-phase liquid, embodiments of this invention generally involve an external force or the like as the separation modality. Such can include separating such as magnetically separating at least some of one type of cells from the collection of cells, electrostatically separating at least some of one type of cells from the collection of cells, separating at least some of one type of cells from the collection of cells by electrophoresis, gravimetrically separating at least some of one type of cells from said collection of cells (perhaps with the right particle or such), and separating at least some of one type of cells from the collection of cells by centrifugation. Thus for sperm, the step of separating at least some of said X-bearing sperm and Y-bearing sperm cells can be through action of the sperm cell associatable particles in the fluid combination of sperm cell associatable particles and sperm cells where the particles (to which a desired portion of the cells are bound) are what is moved by the external force to effect the separation and thus the step of separating is through action of the cell or sperm cell associatable particles.
According to the invention sperm cell associatable particles are combined with the collection of sperm cells to optionally establish a fluid combination (4) of sperm cell associatable particles and sperm cells. Similarly, a plurality of sperm cell sex chromosome differentially associatable particles (3) optionally fluidically combined with the collection of sex chromosome differential exhibiting sperm cells may establish a fluid combination of sperm cell sex chromosome differentially associatable particles and sex chromosome differentially exhibiting sperm cells. The particles can be nano sized such as might establish a suspension or even micro sized such as might establish a settleable mixture or such. Here, the process can involve combining cell or sperm cell associatable nanoparticles with the collection of cells or sperm cells to establish a fluid combination of cell or sperm cell associatable nanoparticles and sperm cells which can then be separated.

The sizes can be varied and designed as desired. Using sperm cells as but one example, systems can involve sperm cell associatable nanoparticles, particles can be: sperm cell associatable particles having an mean diameter of between 10nm and 999nm, sperm cell associatable microparticles, sperm cell associatable particles having an mean diameter of between 100nm and 100µm, sperm cell associatable particles having an mean diameter of not more than 1000nm, sperm cell associatable particles having an mean diameter of 670nm, sperm cell associatable particles having an mean diameter at least 100nm, sperm cell associatable particles having an mean diameter at least 300nm, sperm cell associatable particles having an mean diameter at least 500nm, sperm cell associatable particles having an mean diameter at least 600nm.

Particles can be made of a great variety of substances such as: iron oxide particles, glass particles, silica particles (sol-gel), silica with aluminum substitution particles (such as can be employed to increase the negative colloidal charge, especially when it is evaluated at pH below the neutral point perhaps because of their very small size, the surface area of colloidal silica is very high), borosilicate particles, plastic particles, PVP particles, styrofoam, polyvinlypropylene particles, polyvinylpyrrolidone particles, polystyrene particles, melamine particles (polymethylenemelamine nanospheres and microspheres are made from crosslinked melamine and can have some advantages depending on the application compared to polystyrene particles: they can have a higher density (~1.51g/cm³), can be very stable, can be stored indefinitely, can be resuspended in water, do not swell or shrink in most organic solvents, and can be heat resistant up to 300°C. Further, the surface of plain melamine microparticles are often terminated with methylol groups, which could be readily functionalized in a desired manner), PMMA particles, (polymethymethacrylate is a synthetic resin produced from the polymerization of methyl methacrylate, represents a transparent and rigid plastic), polylactide particles (poly or polylactic acid or polylactide is a biodegradable and bioactive thermoplastic aliphatic polyester derived from renewable resources, such as corn starch, cassava roots, chips or starch, or sugarcane that at one time had the second highest consumption volume of any bioplastic of the world), particles bound to polar molecules (so as to change the zeta potential to bind to cells that are positively charged), dextran particles, functionalized surface particles, magnetic or such particles coated with any of the above materials, or functionalized surfaces for all the above particles. These particles can be mobile in the fluid, can be as a packed column perhaps such as micro or micron sized particles used in packed columns, or as an item that can achieve settling in a solution.

Further, the invention may be used for achieving artificial insemination and production of animals using the resultant products. However, the invention does not cover methods for artificial insemination nor the sperm cells, as obtained according to the invention per se nor the production of animals nor the animals per se.
Artificial insemination using semen with this type of preselected sex could have several potential benefits such as: higher production levels with reduced costs, improvement in animal health and welfare, reduction of environmental impact due to the elimination of the unwanted sex before they grow to adulthood, and faster genetic progress. The present invention may provide a more commercially viable technique with advantages such as: (i) capital equipment intensive free, to reduce costs and exclude the need for highly trained technicians; (ii) non-destructive, to avoid any vitality alteration of the separated sperm; (iii) bulk separations to produce more separated sperm in a shorter amount of time.

As mentioned, embodiments can involve processes such as proximally situating a substance in the vicinity of a collection of sperm cells; associating a desired portion of the collection of sperm cells with a substance; and separating types of cells, such as X-bearing sperm cells and Y-bearing sperm cells, through action of the substance. As mentioned, embodiments can establish a packed column, suspension, a mixture, or such. For a packed column, the column may be flooded with a buffer inducing a net negative charge on the surface of the particles. X-bearing sperm would be collected as the effluent using either gravity or pressure to force the fluid through the column. A full range of option are available for a method including at least the steps of claim 1 and involving: suspending sperm cell associatable particles with a collection of sperm cells to create a suspension of sperm cell associatable particles in a collection of sperm cells, combining likely heavier or larger sperm cell associatable particles with a collection of sperm cells to create an unsuspended collection of sperm cell associatable particles in the collection of sperm cells, mixing sperm cell associatable particles with a collection of sperm cells to create a mixture of sperm cell associatable particles in the collection of sperm cells, moving sperm cell associatable particles through a collection of sperm cells, employing a packed column that may provide a sperm cell passageway past said sperm cell associatable particles or sperm cell associatable particles passageway past said sperm cells, and moving a collection of sperm cells through the sperm cell associatable particles.
Larger particles can be employed to specifically avoid establishing a suspension and even forcing the particles to settle out once associated and here cell or perhaps sperm cell associatable particles having a mean diameter at least 1000nm can be used.

The particles, surfaces, or substances can be manufactured with, perhaps coated with, a desired substance so as to be coated sperm cell associatable particles. These coated or otherwise treated particles can include a great variety of substances to suit the particular application, including but not limited to: carboxyl modified silane coated sperm cell associatable particles, carbohydrate coated sperm cell associatable particles, ligand coated sperm cell associatable particles, Sambucus nigra agglutinin (SNA) coated sperm cell associatable particles, Monosaccharide coated sperm cell associatable particles, antibody coated sperm cell associatable particles, polymerase associatable particles, receptor molecule associatable particles, Cas9-type associatable particles, CRISPR-type associatable particles, DNA tag associatable particles, sperm cell differentiatable condition active sperm cell associatable particles. They can be manufactured using known techniques so the steps of combining can involve: combining particles stabilized by pH adjustment with a collection of sperm cells to establish a fluid combination of sperm cell associatable particles and sperm cells, combining a pH particle combination stabilized particle containing fluid with a collection of sperm cells to establish a fluid combination of sperm cell associatable particles and sperm cells, combining a particle concentrated particle fluid with a collection of sperm cells to establish a fluid combination of sperm cell associatable particles and sperm cells, combining a stable particle fluid with a collection of sperm cells to establish a fluid combination of sperm cell associatable particles and sperm cells, combining a stable concentration level particle fluid with a collection of sperm cells to establish a fluid combination of sperm cell associatable particles and sperm cells, combining a particle size-concentration level coordinated stable particle fluid with a collection of sperm cells to establish a fluid combination of sperm cell associatable particles and sperm cells, combining a 50nm particle size-50%wt solids particle fluid with a collection of sperm cells to establish a fluid combination of sperm cell associatable particles and sperm cells, combining a 10nm particle size-30%wt solids particle fluid with a collection of sperm cells to establish a fluid combination of sperm cell associatable particles and sperm cells. In manufacture, the colloidal or other suspension can be stabilized by pH adjustment and then concentrated, perhaps by evaporation. The maximum concentration obtainable can depend on the particle size. For example, 50 nm particles can be concentrated to greater than 50 wt% solids while 10 nm particles can only be concentrated to approximately 30 wt% solids before the suspension becomes too unstable.

In accordance with the invention, targeted, and capacitated cells labeled with 'charged' magnetic particles and subjected to magnetic cell separation in an 'open' (columnless) magnetic system can be removed more efficiently and in greater numbers per time unit compared to flow cytometry. Magnetic cell separation requires no internal operating pressure or if pressurized, a lower internal operating pressure and the stream of fluid containing the cells does not have to be broken into cell damaging droplets as that for flow cytometry. Further, the sheath fluid required for flow cytometry is generally a salt-based, lipo-protein deficient physiological medium. Magnetic cell separation can allow some cells, such as sperm in but one, non-limiting example, to be bathed in nutrient-rich buffers that may promote and prolong cell viability during the separation procedure. Further, for some embodiments and applications, it may also be beneficial to separate the cells in a buffer, at a temperature, or cause situations that otherwise might render them less mobile or even immobile for a time so that normal swimming does not impact a desired separation process. In other cell separation applications, embodiments of the present invention can be used to magnetically label and ultimately remove capacitated sperm and/or Y sperm through magnetic cell separation procedures. The resultant desired sub-population of harvested cells can be selected for viable X sperm as well as for specific cellular attributes.

As mentioned throughout, the present invention can be adapted in a broad number of ways. As just one type of the many examples of adaptation of these teachings, even the previously tried but unsuccessful Percoll method might be an adaptation. As should be understood, Percoll might present another type of associationally active element. It may not have worked before because Percoll with the buffer in it is a negatively charged environment. It may have removed the dead and dying sperm but not altered sex ratio unless it were applied in a manner according to the present invention whereby the sperm were appropriately or perhaps forcibly capacitated such as with a capacitation agent to increase the pH and to affirmatively establish the differential effect.
With this, it is likely that one could even adapt Percoll to separate X from Y.

As mentioned above, the viability of the cells can be important - especially for sperm cells. For this aspect, embodiments of the invention can involve selectively impacting substantially only one type of cells from said collection of cells while leaving the other type of cells from said collection of cells substantially unimpacted, such as, for sperm cells, selectively impacting substantially only one type of sex chromosome bearing sperm cells from said collection of sperm cells while leaving the other type of sex chromosome bearing sperm cells from said collection of sperm cells substantially unimpacted, and acting on at least a portion of said only one type of sex chromosome bearing sperm cells that have been selectively impacted. By acting on only one type of cell (e.g., unlike the flow cytometry act of staining both types), the other, perhaps desired type of cell is nearly totally unimpacted through processes that cause no substantial effect on one type of cells from the collection of cells. Those unaffected cells can only have natural effects and by the act of subjecting that one type of cells from the collection of cells to only natural effects they exist in a better condition. This can be done by avoiding actions on or condition for such cells including but not limited to steps such as: avoiding associating any unnatural substance with one type of cells from the collection of cells perhaps, avoiding staining one type of cells from the collection of cells, avoiding subjecting one type of cells from the collection of cells to any non-naturally occurring external forces, non-cell motility separating at least some of one type of cells from the collection of cells, avoiding hyperactivity in one type of cells from the collection of cells such as can be caused by the acrosome reaction of the like, avoiding any physical activity for one type of cells from the collection of cells so they are not stressed, tired, or weakened. It can also be done by providing a no unnatural substance for one type of cells collection of cells (18), a no stain collection of cells (19), a no hyperactivity collection of cells (20), a no physical activity collection of cells (21).

**In** considering the example of embodiments involving avoiding subjecting one type of cells from the collection of cells to any non-naturally occurring external forces, it can be interesting to note that if designed with some of the disclosed separation modalities, the step of separating at least some of one type of cells from the collection of cells can occur while acting substantially passively with respect to another type of the cells. The external force or external force separation modality acts on the type of cells associated with particles, not the other type of cells and so when external force separating at least some of one type of cells from the collection of cells, the other type of cells is substantially undisturbed. Once acted upon to achieve separation, embodiments can capture a desired (or for that matter in the overall use scheme an undesired) type of cells in the collection of cells or can provide a desired type of cell capture element. All of these aspects can contribute to eventually providing, as an end result of separation, cells that are more viable which is certainly a desire for sperm cells to be used in fertilization processes.

This feature of embodiments of the invention can be critical to ultimate application of the invention. Using sperm cells, the initial collection of cells may be established with both the X-bearing and Y-bearing sperm cells in the collection prior to separation as being functionally viable X-bearing and Y-bearing sperm cells. These cells should be functionally viable cells that are usable in practical application for fertilization processes be them AI, IVF, or otherwise. For artificial insemination purposes, both going in and coming out of the overall separation system, the desired cells should be functionally viable cells usable in practical application for artificial insemination processes. Even the removed cells should be cells, at least prior to being associated and separated, that are or were functionally viable cells usable in practical application for artificial insemination processes. Allowing capacitation to proceed too far, as one example, as when in the acrosome reaction, can destroy viability in the sense that the cells will have substantial losses, and so embodiments of the invention can require that both the X-bearing and Y-bearing sperm cells in the collection prior to separation be sperm cells in a state where they are practically usable for fertilization without substantial loss of those cells. Thus, embodiments can include using pre-acrosome reaction initiation sperm cells. Other embodiments of the invention can, of course, be applied to remove non-viable cells and so embodiments can include dying or functionally impaired cells, perhaps such as sperm cells, in novel ways. However, using viable cells in general can include having the cells, perhaps sperm cells, in a state where they are or would be viable in the senses stated above: after overnight storage, after shipping in a natural state, after freezing, shipping and thawing, after having been frozen, after having been frozen and then thawed, after at least 8 hours for cells held in an unfrozen state in seminal plasma, after at least 16 hours for cells held in an unfrozen state in seminal plasma, after at least 24 hours for cells held in an unfrozen state in seminal plasma, after at least 30 minutes after thawing for cells frozen and then thawed, after at least 45 minutes after thawing for cells frozen and then thawed, after at least 1 hour after thawing for cells frozen and then thawed, after at least 2 hours after thawing for cells frozen and then thawed, cells that remain practically usable for fertilization without a loss of more than 20% of such cells, cells that remain practically usable for fertilization without a loss of more than 30% of such cells, cells that remain practically usable for fertilization without a loss of more than 40% of such cells.

As mentioned, control and repeatability of embodiments, and perhaps viability, can be accomplished by timing such as of a differential transition effect for the collection of cells and for sperm cells experiencing a capacitation effect, values can include using and acting on: sperm cells less than 90 minutes after having been subjected to a capacitation change effect, sperm cells less than 120 minutes after having been subjected to a capacitation change effect, sperm cells less than 150 minutes after having been subjected to a capacitation change effect, sperm cells less than 180 minutes after having been subjected to a capacitation change effect, sperm cells less than 210 minutes after having been subjected to a capacitation change effect, sperm cells less than 90 minutes after having been subjected to a heparin activation, sperm cells less than 120 minutes after having been subjected to a heparin activation, sperm cells less than 150 minutes after having been subjected to a heparin activation, sperm cells less than 180 minutes after having been subjected to a heparin activation, sperm cells less than 210 minutes after having been subjected to a heparin activation, sperm cells in a state of from 180-240 minutes after having been subjected to a heparin, activation.
Further, to aid in assuring the cells are as viable as possible, embodiments can involve maintaining the sperm cells, in a nurturing environment throughout all or mostly all of the steps of the separation system. In this way, methods can provide one or more nurturing environments (8) for said cells that exist in all aspects of the system. This can involve avoiding subjecting the cells to buffers, conditions (e.g., for sperm cells even isolating those cells from the collection such as in flow cytometry), or other influences that are less than optimal, not generally or universally accepted as safe or non-detrimental, or perhaps just undesired by users. In this regard it may be noted that for sperm cells, using heparin - at least at the initial stages of capacitation such as with pre-acrosome reaction initiation sperm cells - is generally accepted as non-detrimental as merely causing the natural process of capacitation at controlled times external to a natural fertilization process. Nurturing environments can involve including a protein source mixed with the sperm cells throughout all or most of the steps or one or more protein sources (9) for cells that exist in all aspects of the disclosed methods.
With this understanding, the current process defined in claim 1 can preferably involve some combination of the following steps: 1) obtaining a sample; 2) incubating the sample in an appropriate buffer to induce the claimed differential effect; 3) suspending associationally active particles in a similar or perhaps identical buffer; 4) mixing an aliquot of suspended particles with suspended cells at desired ratios; 5) incubating the mixture for a desired period of time; 6) subjecting the mixture to the claimed separation modality for a set period of time; and 7) separating, perhaps by removing, a portion of the mixture so as to yield a desired resultant selected portion of the total. In addition, a more detailed embodiment can include aspects such as: a) pre- incubation of Y sperm and X sperm in the Sp-TALP-H buffer perhaps such as with 20 ug/ml for 3 hours; b) resuspension of the cells perhaps such as in 4 ml of Sp-TALP-H buffer with 20 ug/ml heparin and BSA; c) achieving a desired cell concentration perhaps such as 160 million cells/ml; removing from dH₂O, magnetically collected particles; d) resuspension of iron oxide nanoparticles perhaps such as in 1 ml of Sp-TALP-H buffer with 20 ug/ml of heparin; e) adding an appropriate amount of particles to an aliquot perhaps such as so that the total of the particles added equals 100 µl of the total volume; incubating the semen and particles perhaps such as for 20 minutes at room temperature; f) placing the mixture in front of a magnet to potentially induce a desired net zeta charge and also to allow migration perhaps such as for three minutes; g) aspirating out the nonmagnetic fraction; and h) producing a fraction suitable for desired purposes perhaps such as for artificial insemination. Of course, additional optional steps disclosed above throughout this disclosure can be used including but not limited to using and even thawing a frozen semen amount to obtain the sample prior to achieving separation on the thawed sample.
Using just the subset of cells that are sperm cells as but one example, embodiments can be designed and adapted to involve selecting sperm cells that have never been frozen for the collection of sperm cells, selecting sperm cells that have been frozen and then thawed for the collection of sperm cells, selecting human sperm cells for the collection of sperm cells, selecting non-human sperm cells for the collection of sperm cells, selecting bovine sperm cells for the collection of sperm cells, selecting porcine sperm cells for the collection of sperm cells, separating at least some X-bearing sperm cells and Y-bearing sperm cells. Particular embodiments can involve using: sperm cells, X-bearing sperm cells and Y-bearing sperm cells, X-bearing sperm cells and Y-bearing sperm cells for artificial insemination use, dying or functionally impaired sperm cells, and even dying or functionally impaired cells in general. As mentioned above, all of these artificial insemination methods are not part of the invention.

One aspect where the invention provides a long desired, but never repeatable or enabled to be achieved, feature is that of providing a bulk separation process especially for sexed sperm cells. For sexed sperm cells, by providing the advantages of speed of separation and cost of separation, bulk separating the cells has the potential of literally changing the industry as compared to the only practical, repeatable, enabled sex separation process, namely, flow cytometry processing which is done one-by-one and with rather harsh treatment of the sperm cells. As a bulk separation process, embodiments of the invention can involve acting on at least some of the collection of sperm cells in a manner that allows bulk separating at least some of the X-bearing sperm cells and Y-bearing sperm cells based upon said sperm cell sex chromosome related differential effect. Further, such as for the viability concerns mentioned above, the act of bulk separating cells can be accomplished while the cells are still in the collection of cells. Further, bulk separating the cells can involve separating the cells more than one-at-a-time, simultaneously separating a significant quantity of said cells in the collection of cells, simultaneously separating the majority of the desired type of said cells in the collection of cells, simultaneously separating substantially all of the desired type of said cells in the collection of cells, simultaneously separating at least ten thousand of the cells at a time, a more than one-at-a-time separation modality, a simultaneous significant quantity cell separation modality, a simultaneous majority of the desired type of cell separation modality, a simultaneous substantially all of the desired type of cell separation modality, an at least ten thousand of said cells at a time simultaneous cell separation modality.

These bulk processes can even be accomplished to repeatably to separate at remarkable purities both for the new existence of a bulk process and even as compared to the flow cytometry-based processes. These can include purities such as: separating substantially all of a desired type of said cells in said collection of cells, separating substantially all of a desired type of said cells in said collection of cells, separating at least 70% of a desired type of said cells in said collection of cells, separating at least 80% of a desired type of said cells in said collection of cells, separating at least 90% of a desired type of said cells in said collection of cells, separating at least 95% of a desired type of said cells in said collection of cells, separating at least 97% of a desired type of said cells in said collection of cells, separating at least about 98% of a desired type of said cells in said collection of cells, separating at least 99% of a desired type of said cells in said collection of cells, separating so as to leave no appreciable viable cells of a desired type of said cells in said collection of cells, separating at flow cytometry achievable levels of purity.

As mentioned, gravimetric processes can be used for the step of separating the cells. This will involve combining cell associatable particles with the collection of cells to preferably establish a fluid combination of cell associatable particles and sperm cells, associating a desired portion of the cells with the cell associatable particles, and gravimetrically separating at least some of the cells through action of the cell associatable particles. As can be appreciated, embodiments can involve gravitationally separating at least some of one type of cells from the collection of cells. It can also include enhanced force separating at least some of one type of cells from the collection of cells, perhaps such as or also, centrifugationally separating at least some of one type of cells from the collection of cells, and separating at least some of the cells by centrifugation. Such processes can involve using cell associatable microparticles, a gravimetric separation modality (7) and gravimetrically separating with a gravimetric force greater than any cell motility force, a greater than cell motility effect gravimetric cell separation modality, and even cell associatable particles having a mean diameter at least 1000nm so as to not be suspended but to more likely settle out or be gravimetrically forced out of the collection of cells.

## Claims

1. A method for separation of X-bearing sperm cells and Y-bearing sperm cells within a collection of sperm cells, comprising:
triggering capacitation to induce at least some of said Y-bearing sperm cells to exhibit a differential surface charge relative to at least some of said X-bearing sperm cells; and
separating at least some of said X-bearing sperm cells from at least some of said Y-bearing sperm cells through action of sperm cell associatable particles which associate with said Y-bearing sperm cells via said differential surface charge.

2. The method of claim 1, further comprising triggering said capacitation by exposing said sperm cells to heparin.

3. The method of claim 1, further comprising triggering said capacitation by exposing said sperm cells to caffeine.

4. The method of claim 1, further comprising triggering said capacitation by exposing said sperm cells to a pH-altering buffer.

5. The method of claim 1, further comprising exposing said sperm cells to a capacitation trigger until an increase in pH of at least about 0.36 is exhibited.

6. The method of claim 1, further comprising inducing said Y-bearing sperm cells to exhibit a more positive surface charge.

7. The method of claim 1, further comprising magnetically separating at least some of said X-bearing sperm cells from at least some of said Y-bearing sperm cells.

8. The method of claim 1, further comprising selectively impacting substantially only said Y-bearing sperm cells while leaving said X-bearing sperm cells substantially unimpacted.

9. The method of claim 1, wherein the capacitation-triggered sperm cells comprise pre-acrosome reaction initiation sperm cells.

10. The method of claim 1, further comprising inducing cleavage of sialic acid groups on the surface of at least some of said Y-bearing sperm cells.

11. The method of claim 1, further comprising combining said sperm cell associatable particles with said collection of sperm cells to establish a fluid combination.

12. The method of claim 1, further comprising quenching said capacitation.

13. The method of claim 1, wherein said X-bearing sperm cells are usable for fertilization following said separation.

14. The method of claim 1, wherein said X-bearing sperm cells are usable for artificial insemination following said separation.

## Patentansprüche

1. Verfahren zur Trennung von X-Spermazellen und Y-Spermazellen in einer Sammlung von Spermazellen, umfassend:
Auslösen einer Kapazitation für das Veranlassen, dass wenigstens einige der Y-Spermazellen eine differentielle Oberflächenladung relativ zu wenigstens einigen der X-Spermazellen aufweisen, und
Trennen wenigstens einiger der X- Spermazellen von wenigstens einigen der Y-Spermazellen durch eine Wirkung von mit Spermazellen assoziierbaren Partikeln, die sich mit den Y-Spermazellen aufgrund der differentiellen Oberflächenladung assoziieren.

2. Verfahren nach Anspruch 1, das weiterhin das Auslösen der Kapazitation durch das Aussetzen der Spermazellen an Heparin umfasst.

3. Verfahren nach Anspruch 1, das weiterhin das Auslösen der Kapazitation durch das Aussetzen der Spermazellen an Koffein umfasst.

4. Verfahren nach Anspruch 1, das weiterhin das Auslösen der Kapazitation durch das Aussetzen der Spermazellen an einen pH-Wert-verändernden Puffer umfasst.

5. Verfahren nach Anspruch 1, das weiterhin das Aussetzen der Spermazellen an einen Kapazitationsauslöser, bis sie eine Erhöhung des pH-Werts um wenigstens 0,36 aufweisen, umfasst.

6. Verfahren nach Anspruch 1, das weiterhin das Veranlassen, dass Y-Spermazellen eine positivere Oberflächenladung aufweisen, umfasst.

7. Verfahren nach Anspruch 1, das weiterhin das magnetische Trennen wenigstens einiger der X-Spermazellen von wenigstens einigen der Y-Spermazellen umfasst.

8. Verfahren nach Anspruch 1, das weiterhin das wahlweise Beeinflussen nur der Y-Spermazellen, während die X-Spermazellen im Wesentlichen unbeeinflusst bleiben, umfasst.

9. Verfahren nach Anspruch 1, wobei die Spermazellen mit einer ausgelösten Kapazitation eine Prä-Akrosomreaktion einleitende Spermazellen umfassen.

10. Verfahren nach Anspruch 1, das weiterhin das Veranlassen einer Spaltung von Sialinsäuregruppen an der Oberfläche wenigstens einiger der Y-Spermazellen umfasst.

11. Verfahren nach Anspruch 1, das weiterhin das Kombinieren der mit Spermazellen assoziierbaren Partikeln mit der Sammlung von Spermazellen für das Herstellen einer Fluidkombination umfasst.

12. Verfahren nach Anspruch 1, das weiterhin das Beenden der Kapazitation umfasst.

13. Verfahren nach Anspruch 1, wobei die X-Spermazellen für eine auf die Trennung folgende Befruchtung verwendet werden können.

14. Verfahren nach Anspruch 1, wobei die X-Spermazellen für eine auf die Trennung folgende künstliche Insemination verwendet werden können.

## Revendications

1. Procédé de séparation de spermatozoïdes portant un chromosome X et de spermatozoïdes portant un chromosome Y au sein d'un ensemble de spermatozoïdes, comprenant les étapes consistant à :
déclencher la capacitation pour induire la présentation par au moins certains desdits spermatozoïdes portant un chromosome Y d'une charge de surface différentielle par rapport à au moins certains desdits spermatozoïdes portant un chromosome X ; et
séparer au moins certains desdits spermatozoïdes portant un chromosome X d'au moins certains desdits spermatozoïdes portant un chromosome Y par l'action de particules pouvant être associées à des spermatozoïdes qui s'associent auxdits spermatozoïdes portant un chromosome Y par l'intermédiaire de ladite charge de surface différentielle.

2. Procédé selon la revendication 1, comprenant en outre l'étape consistant à déclencher ladite capacitation en exposant lesdits spermatozoïdes à de l'héparine.

3. Procédé selon la revendication 1, comprenant en outre l'étape consistant à déclencher ladite capacitation en exposant lesdits spermatozoïdes à de la caféine.

4. Procédé selon la revendication 1, comprenant en outre l'étape consistant à déclencher ladite capacitation en exposant lesdits spermatozoïdes à un tampon modifiant le pH.

5. Procédé selon la revendication 1, comprenant en outre lesdits spermatozoïdes à un déclencheur de capacitation jusqu'à ce qu'une augmentation de pH d'au moins environ 0,36 soit observée.

6. Procédé selon la revendication 1, comprenant en outre l'étape consistant à induire la présentation par lesdits spermatozoïdes portant un chromosome Y d'une charge de surface plus positive.

7. Procédé selon la revendication 1, comprenant en outre l'étape consistant à séparer magnétiquement au moins certains desdits spermatozoïdes portant un chromosome X d'au moins certains desdits spermatozoïdes portant un chromosome Y.

8. Procédé selon la revendication 1, comprenant en outre l'étape consistant à affecter de manière sélective essentiellement uniquement lesdits spermatozoïdes portant un chromosome Y tout en n'affectant essentiellement pas lesdits spermatozoïdes portant un chromosome X.

9. Procédé selon la revendication 1, dans lequel les spermatozoïdes déclenchés par la capacitation comprennent des spermatozoïdes à initiation de réaction pré-acrosome.

10. Procédé selon la revendication 1, comprenant en outre l'étape consistant à induire le clivage des groupements acide sialique à la surface d'au moins certains desdits spermatozoïdes portant un chromosome Y.

11. Procédé selon la revendication 1, comprenant en outre l'étape consistant à combiner lesdites particules pouvant être associées aux spermatozoïdes audit ensemble de spermatozoïdes pour établir une combinaison fluide.

12. Procédé selon la revendication 1, comprenant en outre l'étape consistant à éteindre ladite capacitation.

13. Procédé selon la revendication 1, dans lequel lesdits spermatozoïdes portant un chromosome X peuvent être utilisés pour la fertilisation après ladite séparation.

14. Procédé selon la revendication 1, dans lequel lesdits spermatozoïdes portant un chromosome X peuvent être utilisés pour l'insémination artificielle après ladite séparation.
